# EUROPEAN PATENT SPECIFICATION

(11) **EP 1 711 604 B1**
(45) Date of publication and mention of the grant of the patent: **05.10.2011**
(21) Application number: 05700261.0
(22) Date of filing: 10.01.2005
(51) Int. Cl.: C12N 15/11, C07K 7/06, C07K 16/16, A23L 1/29, C07K 14/415, C12N 15/29, G01N 33/02, G01N 33/48, G01N 33/564

(54) **DIABETOGENIC EPITOPES**
DIABETOGENE EPITOPE
EPITOPES DIABETOGENES

(30) Priority: 09.01.2004 US 535278 P
(43) Date of publication of application: 18.10.2006
(73) Proprietor: Ottawa Health Research Institute, Ottawa, Ontario K1Y 4E9 (CA)
(72) Inventor: SCOTT, Fraser, W., Ottawa, Ontario K1Y 1A8 (CA); MACFARLANE, Amanda, Ottawa, Ontario K1S 5B7 (CA); BURGHARDT, Karolina, Orleans, Ontario K4A 1R1 (CA); MOJIBIAN, Majid, Ottawa, Ontario K1V 7T2 (CA)
(74) Representative: Larfeldt, Helene
(86) International application number: PCT/CA2005/000025
(87) International publication number: WO 2005/066345

(56) References cited:
- WO-A2-01/25793
- US-A1- 2004 216 190
- DATABASE EMBL/GENBANK/DDBJ 6 August 2002 (2002-08-06), ALTENBACH, S. ET AL.: "WHE3558_A12_B24ZS Wheat developing grains cDNA library Triticum aestivum" XP002470863 retrieved from EBI Database accession no. BQ804720
- OKITA TW. ET AL.: 'Evolution and heterogeneity of the alpha-beta-type and gama ty' JOURNAL OF BIOLOGICAL CHEMISTRY. vol. 260, no. 13, 05 July 1985, pages 8203 - 8213, XP000982597
- DATABASE GENBANK [Online] 27 April 1993 LITTS J.C. ET AL.: 'Genomic nucleotide sequence of a triticum aestivum 7S', XP008094567 Database accession no. M81719 & LITTS JC. ET AL: 'Genomic nucleotide sequence of a triticum aestivu,m 7S globuli' BETHESDA, MD: NATIONAL CENTER FOR BIOTECHNOLOGY INFORMATION, [Online] 27 April 1993, Retrieved from the Internet: <URL:http://www.ncbi.nlm.nih.gov/entrez/vie wer.fcgi?db=nucleotide>
- OSMAN A.A. ET AL.: 'A monoclonal antibody that recognizes a potencial coeliac-' EUROPEAN JOURNAL OF GASTROENTEROLOGY AND HEPATOLOGY. vol. 13, no. 10, October 2001, pages 1189 - 1193, XP008094670
- GIL GARCIA JR. ET AL.: 'Development of a monoclonal antibody capable of detecting' HYBRIDOMAS AND HYBRIDOMICS. vol. 22, no. 6, December 2003, pages 383 - 388, XP008094686
- MACFARLANE A.J. ET AL.: 'Atype 1 diabates-related protein from wheat (triticum aestivum' JOURNAL OF BIOLOGICAL CHEMISTRY. vol. 278, no. 1, 03 January 2003, pages 54 - 63, XP008095160

### SEQUENCE LISTING

<110> Ottawa Health Research Institute
<120> Diabetogenic Epitopes
<130> 034205.003
<150> PCT/CA05/00025
   <151> 2005-01-10
<150> US 60/535,278
   <151> 2004-01-09
<160> 52
<170> Patent In version 3.3
<210> 1
   <211> 10
   <212> PRT
   <213> Artificial
<220>
   <223> Diabetogenic epitope from gliadin protein isoforms or Glb1 based on wheat protein
<400> 1
   Glu Glu Gln Leu Arg Glu Leu Arg Arg Gln
   1               5                   10
<210> 2
   <211> 9
   <212> PRT
   <213> Unknown
<220>
   <223> Tryptic peptide of wheat storage globulin
<400> 2
   Val Ala Ile Met Glu Val Asn Pro Arg
   1               5
<210> 3
   <211> 2018
   <212> DNA
   <213> Unknown
<220>
   <223> Wheat gene
<400> 3
   atggcgacca gaggcagagc aaccatccct ctcctcttcc tcctgggcac aagccttctc      60
   ttcgccgcgg ctgtttcggc ctcccatgac gaggaggagg acaggcgcgg tgggcgctcg     120
   cttcagcggt gcgtgcagcg gtgccagcag gaccggccgc ggtactctca tgcccggtgc     180
   gtgcaggagt gccgggacga ccagcagcag cacggaaggc acgagcagga ggagcagggc    240
   cgcgggcatg gccggcacgg cgagggggag cgtgaggagg agcagggccg tggccgtggg    300
   cggcgcggcc agggagagcg tgaggaggag cagggccgtg gacgtgggcg gcgcggcgag    360
   ggagagcgtg atgaggagca cggggatggc cggcggccgt acgtgttcgg cccgcgcagc    420
   ttccgccgca tcatccggag cgaccacggg ttcgtcaagg cccttcgccc gttcgacgaa    480
   gtgtccaggc tcctccgggg catcaggaac taccgtgtcg ccatcatgga ggtgaacccg    540
   cgcgcgttcg tcgtgccggg actcacggac gcagacggcg tcggctacgt cgctcaaggc    600
   gagggggtgc tgacggtgat cgagaacggc gagaagcggt cctacaccgt caggcaaggc    660
   gatgtgatcg tggcgccggc ggggtccatc atgcacctgg ccaacaccga cggccggagg    720
   aagctggtca tcgccaagat tctccacacc atctccgtcc ccggcaagtt ccagtatttc    780
   tcggccaagc ctctcctcgc tagtttgagc aaacgcgtgc tcacagcggc gttaaagacc    840
   tcggatgagc ggctgggtag tctcttgggc agccgccaag gcaaggagga ggaggagaag    900
   tccatctcca tcgtccgcgc gtcagaggag cagctccgcg agctgcgtcg ccaggcgtcc    960
   gagggtgacc agggccacca ctggcctctc cccccgttcc gcggcgactc gcgcgacacc   1020
   ttcaacctcc tggagcagcg ccccaagatc gccaaccgcc atggccgcct ctacgaggcc   1080
   gacgcccgta gcttccacgc cctcgcccaa cacgacgtcc gcgtcgccgt ggccaacatc   1140
   acgccgggtt ctatgaccgc gccctacctg aacacccagt cgttcaagct cgccgtcgtg   1200
   ctggaaggcg agggcgaggt ggagatcgtc tgcccgcacc tcggccgcga cagcgagcgc   1260
   cgcgagcaag agcacggcaa gggcaggtgg aggagcgagg aagaggagga cgaccggcgg   1320
   cagcaacgcc gacgcgggtc cggctccgag tcggaggagg agcaggacca gcagaggtac   1380
   gagacggtcc gcgcgcgggt gtcgcgcggc tcggcgttcg tggtgccccc cggccacccg   1440
   gtggtggaga tcgcctcgtc ccgcggcagc agcaacctcc aggtggtgtg cttcgagatc   1500
   aacgccgaga ggaacgagcg ggtgtggctc gccgggagga acaacgtgat cgccaagctg   1560
   gacgaccccg cccaggagct cgccttcggc aggcccgcga gggaggtgca ggaggtgttc   1620
   cgcgccaagg atcagcagga cgagggcttc gtcgccggac ccgagcagca gcaggagcat   1680
   gagcgcgggg accgccgccg tggtgaccgc gggcgcggcg acgaagccgt ggaggcgttc   1740
   ctgaggatgg caaccgccgc gctctgaggc ggcaaggccg ctgttgttaa gtgaatgtgt   1800
   gagctggagc ccgtgccatt tgagagctga acttgtatgt gtgtgtaagt ttgtcagtac   1860
   gcgggagtag cataaataag tcgtggcacg ggctcagtac gatgatgtaa gttgcgtacc   1920
   taccttctac caaggcatgc atgcccaaca taaataaaca caagggcgtt gcgcctcttt   1980
   ttcagtaaaa aaaaaaaaaa aaaaaaaaaa aaaaaaaa 2018
<210> 4
   <211> 588
   <212> PRT
   <213> Unknown
<220>
   <223> WP5212 wheat protein sequence
<400> 4
   Met Ala Thr Arg Gly Arg Ala Thr Ile Pro Leu Leu Phe Leu Leu Gly
   1               5                   10                  15
   Thr Ser Leu Leu Phe Ala Ala Ala Val Ser Ala Ser His Asp Glu Glu
               20                  25                  30
   Glu Asp Arg Arg Gly Gly Arg Ser Leu Gln Arg Cys Val Gln Arg Cys
           35                  40                  45
   Gln Gln Asp Arg Pro Arg Tyr Ser His Ala Arg Cys Val Gln Glu Cys
       50                  55                  60
   Arg Asp Asp Gln Gln Gln His Gly Arg His Glu Gln Glu Glu Gln Gly
   65                  70                  75                  80
   Arg Gly His Gly Arg His Gly Glu Gly Glu Arg Glu Glu Glu Gln Gly
                   85                  90                  95
   Arg Gly Arg Gly Arg Arg Gly Gln Gly Glu Arg Glu Glu Glu Gln Gly
               100                 105                 110
   Arg Gly Arg Gly Arg Arg Gly Glu Gly Glu Arg Asp Glu Glu His Gly
           115                 120                 125
   Asp Gly Arg Arg Pro Tyr Val Phe Gly Pro Arg Ser Phe Arg Arg Ile
       130                 135                 140
   Ile Arg Ser Asp His Gly Phe Val Lys Ala Leu Arg Pro Phe Asp Glu
   145                 150                 155                 160
   Val Ser Arg Leu Leu Arg Gly Ile Arg Asn Tyr Arg Val Ala Ile Met
                   165                 170                 175
   Glu Val Asn Pro Arg Ala Phe Val Val Pro Gly Leu Thr Asp Ala Asp
               180                 185                 190
   Gly Val Gly Tyr Val Ala Gln Gly Glu Gly Val Leu Thr Val Ile Glu
           195                 200                 205
   Asn Gly Glu Lys Arg Ser Tyr Thr Val Arg Gln Gly Asp Val Ile Val
       210                 215                 220
   Ala Pro Ala Gly Ser Ile Met His Leu Ala Asn Thr Asp Gly Arg Arg
   225                 230                 235                 240
   Lys Leu Val Ile Ala Lys Ile Leu His Thr Ile Ser Val Pro Gly Lys
                   245                 250                 255
   Phe Gln Tyr Phe Ser Ala Lys Pro Leu Leu Ala Ser Leu Ser Lys Arg
               260                 265                 270
   Val Leu Thr Ala Ala Leu Lys Thr Ser Asp Glu Arg Leu Gly Ser Leu
           275                 280                 285
   Leu Gly Ser Arg Gln Gly Lys Glu Glu Glu Glu Lys Ser Ile Ser Ile
       290                 295                 300
   Val Arg Ala Ser Glu Glu Gln Leu Arg Glu Leu Arg Arg Gln Ala Ser
   305                 310                 315                 320
   Glu Gly Asp Gln Gly His His Trp Pro Leu Pro Pro Phe Arg Gly Asp
                   325                 330                 335
   Ser Arg Asp Thr Phe Asn Leu Leu Glu Gln Arg Pro Lys Ile Ala Asn
               340                 345                 350
   Arg His Gly Arg Leu Tyr Glu Ala Asp Ala Arg Ser Phe His Ala Leu
           355                 360                 365
   Ala Gln His Asp Val Arg Val Ala Val Ala Asn Ile Thr Pro Gly Ser
       370                 375                 380
   Met Thr Ala Pro Tyr Leu Asn Thr Gln Ser Phe Lys Leu Ala Val Val
   385                 390                 395                 400
   Leu Glu Gly Glu Gly Glu Val Glu Ile Val Cys Pro His Leu Gly Arg
                   405                 410                 415
   Asp Ser Glu Arg Arg Glu Gln Glu His Gly Lys Gly Arg Trp Arg Ser
               420                 425                 430
   Glu Glu Glu Glu Asp Asp Arg Arg Gln Gln Arg Arg Arg Gly Ser Gly
               435                 440                 445
   Ser Glu Ser Glu Glu Glu Gln Asp Gln Gln Arg Tyr Glu Thr Val Arg
       450                 455                 460
   Ala Arg Val Ser Arg Gly Ser Ala Phe Val Val Pro Pro Gly His Pro
   465                 470                 475                 480
   Val Val Glu Ile Ala Ser Ser Arg Gly Ser Ser Asn Leu Gln Val Val
                   485                 490                 495
   Cys Phe Glu Ile Asn Ala Glu Arg Asn Glu Arg Val Trp Leu Ala Gly
               500                 505                 510
   Arg Asn Asn Val Ile Ala Lys Leu Asp Asp Pro Ala Gln Glu Leu Ala
           515                 520                 525
   Phe Gly Arg Pro Ala Arg Glu Val Gln Glu Val Phe Arg Ala Lys Asp
       530                 535                 540
   Gln Gln Asp Glu Gly Phe Val Ala Gly Pro Glu Gln Gln Gln Glu His
   545                 550                 555                 560
   Glu Arg Gly Asp Arg Arg Arg Gly Asp Arg Gly Arg Gly Asp Glu Ala
                   565                 570                 575
   Val Glu Ala Phe Leu Arg Met Ala Thr Ala Ala Leu
               580                 585
<210> 5
   <211> 291
   <212> PRT
   <213> Unknown
<220>
   <223> Alpha/beta-gliadin A-II precursor of wheat protein
<400> 5
   Met Lys Thr Phe Pro Ile Leu Ala Leu Leu Ala Ile Val Ala Thr Thr
   1               5                   10                  15
   Ala Thr Thr Ala Val Arg Val Pro Val Pro Gln Leu Gln Leu Gln Asn
               20                  25                  30
   Pro Ser Gln Gln Gln Pro Gln Glu Gln Val Pro Leu Val Gln Glu Gln
           35                  40                  45
   Gln Phe Gln Gly Gln Gln Gln Pro Phe Pro Pro Gln Gln Pro Tyr Pro
       50                  55                  60
   Gln Pro Gln Pro Phe Pro Ser Gln Gln Pro Tyr Leu Gln Leu Gln Pro
   65                  70                  75                  80
   Phe Pro Gln Pro Gln Leu Pro Tyr Pro Gln Pro Gln Pro Phe Arg Pro
                   85                  90                  95
   Gln Gln Pro Tyr Pro Gln Pro Gln Pro Gln Tyr Ser Gln Pro Gln Gln
               100                 105                 110
   Pro Ile Ser Gln Gln Gln Gln Gln Gln Gln Gln Gln Gln Gln Gln Gln
           115                 120                 125
   Gln Gln Ile Leu Gln Gln Ile Leu Gln Gln Gln Leu Ile Pro Cys Arg
       130                 135                 140
   Asp Val Val Leu Gln Gln His Asn Ile Ala His Gly Ser Ser Gln Val
   145                 150                 155                 160
   Leu Gln Glu Ser Thr Tyr Gln Leu Val Gln Gln Leu Cys Cys Gln Gln
                   165                 170                 175
   Leu Trp Gln Ile Pro Glu Gln Ser Arg Cys Gln Ala Ile His Asn Val
               180                 185                 190
   Val His Ala Ile Ile Leu His Gln Gln His His His His Gln Gln Gln
           195                 200                 205
   Gln Gln Gln Gln Gln Gln Gln Pro Leu Ser Gln Val Ser Phe Gln Gln
       210                 215                 220
   Pro Gln Gln Gln Tyr Pro Ser Gly Gln Gly Phe Phe Gln Pro Ser Gln
   225                 230                 235                 240
   Gln Asn Pro Gln Ala Gln Gly Ser Phe Gln Pro Gln Gln Leu Pro Gln
                   245                 250                 255
   Phe Glu Glu Ile Arg Asn Leu Ala Leu Gln Thr Leu Pro Ala Met Cys
               260                 265                 270
   Asn Val Tyr Ile Pro Pro Tyr Cys Thr Ile Ala Pro Phe Gly Ile Phe
           275                 280                 285
   Gly Thr Asn
   290
<210> 6
   <211> 307
   <212> PRT
   <213> Unknown
<220>
   <223> Alpha/beta-gliadin MM1 precursor of wheat protein
<400> 6
   Met Lys Thr Phe Leu Ile Leu Ala Leu Leu Ala Ile Val Ala Thr Thr
   1               5                   10                  15
   Ala Arg Ile Ala Val Arg Val Pro Val Pro Gln Leu Gln Pro Gln Asn
               20                  25                  30
   Pro Ser Gln Gln Gln Pro Gln Glu Gln Val Pro Leu Val Gln Gln Gln
           35                  40                  45
   Gln Phe Pro Gly Gln Gln Gln Pro Phe Pro Pro Gln Gln Pro Tyr Pro
       50                  55                  60
   Gln Pro Gln Pro Phe Pro Ser Gln Gln Pro Tyr Leu Gln Leu Gln Pro
   65                  70                  75                  80
   Phe Pro Gln Pro Gln Leu Pro Tyr Pro Gln Pro Gln Leu Pro Tyr Pro
                   85                  90                  95
   Gln Pro Gln Leu Pro Tyr Pro Gln Pro Gln Pro Phe Arg Pro Gln Gln
               100                 105                 110
   Pro Tyr Pro Gln Ser Gln Pro Gln Tyr Ser Gln Pro Gln Gln Pro Ile
           115                 120                 125
   Ser Gln Gln Gln Gln Gln Gln Gln Gln Gln Gln Gln Gln Lys Gln Gln
       130                 135                 140
   Gln Gln Gln Gln Gln Gln Ile Leu Gln Gln Ile Leu Gln Gln Gln Leu
   145                 150                 155                 160
   Ile Pro Cys Arg Asp Val Val Leu Gln Gln His Ser Ile Ala Tyr Gly
                   165                 170                 175
   Ser Ser Gln Val Leu Gln Gln Ser Thr Tyr Gln Leu Val Gln Gln Leu
               180                 185                 190
   Cys Cys Gln Gln Leu Trp Gln Ile Pro Glu Gln Ser Arg Cys Gln Ala
           195                 200                 205
   Ile His Asn Val Val His Ala Ile Ile Leu His Gln Gln Gln Gln Gln
       210                 215                 220
   Gln Gln Gln Gln Gln Gln Gln Pro Leu Ser Gln Val Ser Phe Gln Gln
   225                 230                 235                 240
   Pro Gln Gln Gln Tyr Pro Ser Gly Gln Gly Ser Phe Gln Pro Ser Gln
                   245                 250                 255
   Gln Asn Pro Gln Ala Gln Gly Ser Val Gln Pro Gln Gln Leu Pro Gln
               260                 265                 270
   Phe Glu Glu Ile Arg Asn Leu Ala Leu Glu Thr Leu Pro Ala Met Cys
           275                 280                 285
   Asn Val Tyr Ile Pro Pro Tyr Cys Thr Ile Ala Pro Val Gly Ile Phe
       290                 295                 300
   Gly Thr Asn
   305
<210> 7
   <211> 327
   <212> PRT
   <213> Triticum aestivum
<400> 7
   Met Lys Thr Leu Leu Ile Leu Thr Ile Leu Ala Met Ala Ile Thr Ile
   1               5                   10                  15
   Gly Thr Ala Asn Ile Gln Val Asp Pro Ser Gly Gln Val Gln Trp Leu
               20                  25                  30
   Gln Gln Gln Leu Val Pro Gln Leu Gln Gln Pro Leu Ser Gln Gln Pro
           35                  40                  45
   Gln Gln Thr Phe Pro Gln Pro Gln Gln Thr Phe Pro His Gln Pro Gln
       50                  55                  60
   Gln Gln Val Pro Gln Pro Gln Gln Pro Gln Gln Pro Phe Leu Gln Pro
   65                  70                  75                  80
   Gln Gln Pro Phe Pro Gln Gln Pro Gln Gln Pro Phe Pro Gln Thr Gln
                   85                  90                  95
   Gln Pro Gln Gln Pro Phe Pro Gln Gln Pro Gln Gln Pro Phe Pro Gln
               100                 105                 110
   Thr Gln Gln Pro Gln Gln Pro Phe Pro Gln Gln Pro Gln Gln Pro Phe
           115                 120                 125
   Pro Gln Thr Gln Gln Pro Gln Gln Pro Phe Pro Gln Leu Gln Gln Pro
       130                 135                 140
   Gln Gln Pro Phe Pro Gln Pro Gln Gln Gln Leu Pro Gln Pro Gln Gln
   145                 150                 155                 160
   Pro Gln Gln Ser Phe Pro Gln Gln Gln Arg Pro Phe Ile Gln Pro Ser
                   165                 170                 175
   Leu Gln Gln Gln Leu Asn Pro Cys Lys Asn Ile Leu Leu Gln Gln Cys
               180                 185                 190
   Lys Pro Ala Ser Leu Val Ser Ser Leu Trp Ser Ile Ile Trp Pro Gln
           195                 200                 205
   Ser Asp Cys Gln Val Met Arg Gln Gln Cys Cys Gln Gln Leu Ala Gln
       210                 215                 220
   Ile Pro Gln Gln Leu Gln Cys Ala Ala Ile His Ser Val Val His Ser
   225                 230                 235                 240
   Ile Ile Met Gln Gln Gln Gln Gln Gln Gln Gln Gln Gln Gly Met His
                   245                 250                 255
   Ile Phe Leu Pro Leu Ser Gln Gln Gln Gln Val Gly Gln Gly Ser Leu
               260                 265                 270
   Val Gln Gly Gln Gly Ile Ile Gln Pro Gln Gln Pro Ala Gln Leu Glu
           275                 280                 285
   Ala Ile Arg Ser Leu Val Leu Gln Thr Leu Pro Ser Met Cys Asn Val
       290                 295                 300
   Tyr Val Pro Pro Glu Cys Ser Ile Met Arg Ala Pro Phe Ala Ser Ile
   305                 310                 315                 320
   Val Ala Gly Ile Gly Gly Gln
                   325
<210> 8
   <211> 302
   <212> PRT
   <213> Triticum aestivum
<400> 8
   Met Lys Thr Leu Leu Ile Leu Thr Ile Leu Ala Met Ala Thr Thr Ile
   1 5 10 15
   Ala Thr Ala Asn Met Gln Val Asp Pro Ser Gly Gln Val Gln Trp Pro
   20 25 30
   Gln Gln Gln Pro Phe Pro Gln Pro Gln Gln Pro Phe Cys Gln Gln Pro
   35 40 45
   Gln Gln Thr Ile Pro Gln Pro His Gln Thr Phe His His Gln Pro Gln
   50 55 60
   Gln Thr Phe Pro Gln Pro Gln Gln Thr Tyr Pro His Gln Pro Gln Gln
   65 70 75 80
   Gln Phe Pro Gln Thr Gln Gln Pro Gln Gln Pro Phe Pro Gln Pro Gln
   85 90 95
   Gln Thr Phe Pro Gln Gln Pro Gln Leu Pro Phe Pro Gln Gln Pro Gln
   100 105 110
   Gln Pro Phe Pro Gln Pro Gln Gln Pro Gln Gln Pro Phe Pro Gln Ser
   115 120 125
   Gln Gln Pro Gln Gln Pro Phe Pro Gln Pro Gln Gln Gln Phe Pro Gln
   130 135 140
   Pro Gln Gln Pro Gln Gln Ser Phe Pro Gln Gln Gln Gln Pro Ala Ile
   145 150 155 160
   Gln Ser Phe Leu Gln Gln Gln Met Asn Pro Cys Lys Asn Phe Leu Leu
   165 170 175
   Gln Gln Cys Asn His Val Ser Leu Val Ser Ser Leu Val Ser Ile Ile
   180 185 190
   Leu Pro Arg Ser Asp Cys Gln Val Met Gln Gln Gln Cys Cys Gln Gln
   195 200 205
   Leu Ala Gln Ile Pro Gln Gln Leu Gln Cys Ala Ala Ile His Ser Val
   210 215 220
   Ala His Ser Ile Ile Met Gln Gln Glu Gln Gln Gln Gly Val Pro Ile
   225 230 235 240
   Leu Arg Pro Leu Phe Gln Leu Ala Gln Gly Leu Gly Ile Ile Gln Pro
   245 250 255
   Gln Gln Pro Ala Gln Leu Glu Gly Ile Arg Ser Leu Val Leu Lys Thr
   260 265 270
   Leu Pro Thr Met Cys Asn Val Tyr Val Pro Pro Asp Cys Ser Thr Ile
   275 280 285
   Asn Ile Pro Tyr Ala Asn Ile Asp Ala Gly Ile Gly Gly Gln
   290 295 300
<210> 9
   <211> 20
   <212> PRT
   <213> Unknown
<220>
   <223> Diabetogenic epitope homopolymer based on wheat protein
<400> 9
   Glu Glu Gln Leu Arg Glu Leu Arg Arg Gln Glu Glu Gln Leu Arg Glu
   1 5 10 15
   Leu Arg Arg Gln
   20
<210> 10
   <211> 18
   <212> DNA
   <213> Artificial
<220>
   <223> Forward primer for WP5212 wheat gene
<400> 10
   accacgggtt cgtcaagg 18
<210> 11
   <211> 18
   <212> DNA
   <213> Artificial
<220>
   <223> Reverse primer for WP5212 wheat gene
<400> 11
   aacacctcct gcacctcc 18
<210> 12
   <211> 16
   <212> PRT
   <213> Artificial
<220>
   <223> Antigenic WP5212 peptide based on wheat protein
<400> 12
   Cys Arg Asp Thr Phe Asn Leu Leu Glu Gln Arg Pro Lys Ile Ala Asn
   1               5                   10                  15
<210> 13
   <211> 15
   <212> PRT
   <213> Artificial
<220>
   <223> Antigenic WP5212 peptide based on wheat protein
<400> 13
   Arg Gly Asp Glu Ala Val Glu Ala Phe Leu Arg Met Ala Thr Ala
   1               5                   10                  15
<210> 14
   <211> 8
   <212> PRT
   <213> Unknown
<220>
   <223> Tryptic peptide of wheat protein
<400> 14
   Arg Pro Tyr Val Phe Gly Pro Arg
   1               5
<210> 15
   <211> 9
   <212> PRT
   <213> unknown
<220>
   <223> Tryptic peptide of wheat protein
<400> 15
   Val Ala Ile Met Glu Val Asn Pro Arg
   1               5
<210> 16
   <211> 17
   <212> PRT
   <213> unknown
<220>
   <223> Tryptic peptide of wheat protein
<400> 16
   Ala Gln Asp Gln Asp Glu Gly Phe Val Ala Gly Pro Glu Gln Gln Ser
   1               5                   10                  15
   Arg
<210> 17
   <211> 15
   <212> PRT
   <213> unknown
<220>
   <223> Tryptic peptide of wheat protein
<400> 17
   Phe Gln Phe Leu Ser Val Lys Pro Leu Leu Ala Ser Leu Ser Lys
   1               5                   10                  15
<210> 18
   <211> 14
   <212> PRT
   <213> unknown
<220>
   <223> Tryptic peptide of wheat protein
<400> 18
   Gly Ser Glu Ser Glu Ser Glu Glu Glu Glu Glu Gln Gln Arg
   1               5                   10
<210> 19
   <211> 15
   <212> PRT
   <213> unknown
<220>
   <223> Tryptic peptide of wheat protein
<400> 19
   Leu Gly Ser Pro Ala Gln Glu Leu Thr Phe Gly Arg Pro Ala Arg
   1               5                   10                  15
<210> 20
   <211> 11
   <212> PRT
   <213> unknown
<220>
   <223> Tryptic peptide of wheat protein
<400> 20
   Asp Thr Phe Asn Leu Leu Glu Gln Arg Pro Lys
   1               5                   10
<210> 21
   <211> 11
   <212> PRT
   <213> unknown
<220>
   <223> Tryptic peptide of wheat protein
<400> 21
   Ser Phe His Ala Leu Ala Asn Gln Asp Val Arg
   1               5                   10
<210> 22
   <211> 11
   <212> PRT
   <213> unknown
<220>
   <223> Tryptic peptide of wheat protein
<400> 22
   Gly Gly His Ser Leu Gln Gln Cys Val Gln Arg
   1 5 10
<210> 23
   <211> 10
   <212> PRT
   <213> unknown
<220>
   <223> Tryptic peptide of wheat protein
<400> 23
   Ala Leu Arg Pro Phe Asp Gln Val Ser Arg
   1 5 10
<210> 24
   <211> 10
   <212> PRT
   <213> unknown
<220>
   <223> Tryptic peptide of wheat protein
<400> 24
   Ile Ile Gln Ser Asp His Gly Phe Val Arg
   1 5 10
<210> 25
   <211> 9
   <212> PRT
   <213> unknown
<220>
   <223> Tryptic peptide of wheat protein
<400> 25
   His Glu Gln Glu Glu Glu Gln Gly Arg
   1 5
<210> 26
   <211> 10
   <212> PRT
   <213> unknown
<220>
   <223> Tryptic peptide of wheat protein
<400> 26
   Gly Asp Glu Ala Val Glu Thr Phe Leu Arg
   1 5 10
<210> 27
   <211> 8
   <212> PRT
   <213> unknown
<220>
   <223> Tryptic peptide of wheat protein
<400> 27
   Glu Gln Glu Gln Glu Gln Glu Arg
   1 5
<210> 28
   <211> 10
   <212> PRT
   <213> unknown
<220>
   <223> Tryptic peptide of wheat protein
<400> 28
   Ile Leu His Thr Ile Ser Val Pro Gly Lys
   1 5 10
<210> 29
   <211> 8
   <212> PRT
   <213> unknown
<220>
   <223> Tryptic peptide of wheat protein
<400> 29
   Glu Glu Glu Glu Asp Asp Gln Arg
   1 5
<210> 30
   <211> 10
   <212> PRT
   <213> unknown
<220>
   <223> Tryptic peptide of wheat protein
<400> 30
   Glu Ala Ala Glu Gly Gly Gln Gly His Arg
   1               5                   10
<210> 31
   <211> 8
   <212> PRT
   <213> unknown
<220>
   <223> Tryptic peptide of wheat protein
<400> 31
   Asp Asp Gln Gln Gln His Gly Arg
   1               5
<210> 32
   <211> 29
   <212> PRT
   <213> unknown
<220>
   <223> Tryptic peptide of wheat protein
<400> 32
   Ala Thr Ile Pro Leu Leu Phe Leu Leu Gly Thr Ser Leu Leu Phe Ala
   1               5                   10                  15
   Ala Ala Val Ser Ala Ser His Asp Glu Glu Glu Asp Arg
               20                  25
<210> 33
   <211> 31
   <212> PRT
   <213> unknown
<220>
   <223> Tryptic peptide of wheat protein
<400> 33
   Ala Phe Val Val Pro Gly Leu Thr Asp Ala Asp Gly Val Gly Tyr Val
   1 5 10 15
   Ala Gln Gly Glu Gly Val Leu Thr Val Ile Glu Asn Gly Glu Lys
   20 25 30
<210> 34
   <211> 22
   <212> PRT
   <213> unknown
<220>
   <223> Tryptic peptide of wheat protein
<400> 34
   Val Ala Val Ala Asn Ile Thr Pro Gly Ser Met Thr Ala Pro Tyr Leu
   1 5 10 15
   Asn Thr Gln Ser Phe Lys
   20
<210> 35
   <211> 21
   <212> PRT
   <213> unknown
<220>
   <223> Tryptic peptide of wheat protein
<400> 35
   Gln Gly Asp Val Ile Val Ala Pro Ala Gly Ser Ile Met His Leu Ala
   1 5 10 15
   Asn Thr Asp Gly Arg
   20
<210> 36
   <211> 20
   <212> PRT
   <213> unknown
<220>
   <223> Tryptic peptide of wheat protein
<400> 36
   Leu Ala Val Val Leu Glu Gly Glu Gly Glu Val Glu Ile Val Cys Pro
   1 5 10 15
   His Leu Gly Arg
   20
<210> 37
   <211> 19
   <212> PRT
   <213> unknown
<220>
   <223> Tryptic peptide of wheat protein
<400> 37
   Gly Ser Ala Phe Val Val Pro Pro Gly His Pro Val Val Glu Ile Ala
   1 5 10 15
   Ser Ser Arg
<210> 38
   <211> 19
   <212> PRT
   <213> unknown
<220>
   <223> Tryptic peptide of wheat protein
<400> 38
   Asp Gln Gln Asp Glu Gly Phe Val Ala Gly Pro Glu Gln Gln Gln Glu
   1 5 10 15
   His Glu Arg
<210> 39
   <211> 17
   <212> PRT
   <213> unknown
<220>
   <223> Tryptic peptide of wheat protein
<400> 39
   Gln Ala Ser Glu Gly Asp Gln Gly His His Trp Pro Leu Pro Pro Phe
   1 5 10 15
   Arg
<210> 40
   <211> 16
   <212> PRT
   <213> unknown
<220>
   <223> Tryptic peptide of wheat protein
<400> 40
   Gly Ser Ser Asn Leu Gln Val Val Cys Phe Glu Ile Asn Ala Glu Arg
   1 5 10 15
<210> 41
   <211> 15
   <212> PRT
   <213> unknown
<220>
   <223> Tryptic peptide of wheat protein
<400> 41
   Leu Asp Asp Pro Ala Gln Glu Leu Ala Phe Gly Arg Pro Ala Arg
   1 5 10 15
<210> 42
   <211> 15
   <212> PRT
   <213> unknown
<220>
   <223> Tryptic peptide of wheat protein
<400> 42
   Phe Gln Tyr Phe Ser Ala Lys Pro Leu Leu Ala Ser Leu Ser Lys
   1 5 10 15
<210> 43
   <211> 14
   <212> PRT
   <213> unknown
<220>
   <223> Tryptic peptide of wheat protein
<400> 43
   Gly Ser Gly Ser Glu Ser Glu Glu Glu Gln Asp Gln Gln Arg
   1 5 10
<210> 44
   <211> 11
   <212> PRT
   <213> unknown
<220>
   <223> Tryptic peptide of wheat protein
<400> 44
   Ser Phe His Ala Leu Ala Gln His Asp Val Arg
   1 5 10
<210> 45
   <211> 10
   <212> PRT
   <213> unknown
<220>
   <223> Tryptic peptide of wheat protein
<400> 45
   Gly Asp Glu Ala Val Glu Ala Phe Leu Arg
   1 5 10
<210> 46
   <211> 10
   <212> PRT
   <213> unknown
<220>
   <223> Tryptic peptide of wheat protein
<400> 46
   Ala Leu Arg Pro Phe Asp Glu Val Ser Arg
   1 5 10
<210> 47
   <211> 9
   <212> PRT
   <213> unknown
<220>
   <223> Tryptic peptide of wheat protein
<400> 47
   Gly Asp Ser Ser Thr Met Ala Thr Arg
   1 5
<210> 48
   <211> 8
   <212> PRT
   <213> unknown
<220>
   <223> Tryptic peptide of wheat protein
<400> 48
   Ser Glu Glu Glu Glu Asp Asp Arg
   1 5
<210> 49
   <211> 8
   <212> PRT
   <213> unknown
<220>
   <223> Tryptic peptide of wheat protein
<400> 49
   His Glu Gln Glu Glu Gln Gly Arg
   1 5
<210> 50
   <211> 8
   <212> PRT
   <213> unknown
<220>
   <223> Tryptic peptide of wheat protein
<400> 50
   Asp Glu Glu His Gly Asp Gly Arg
   1 5
<210> 51
   <211> 8
   <212> PRT
   <213> unknown
<220>
   <223> Tryptic peptide of wheat protein
<400> 51
   Leu Gly Ser Leu Leu Gly Ser Arg
   1 5
<210> 52
   <211> 7
   <212> PRT
   <213> unknown
<220>
   <223> Tryptic peptide of wheat protein
<400> 52
   Leu Tyr Glu Ala Asp Ala Arg
   1 5

## Description

### FIELD OF INVENTION

The present invention relates to proteins which are antigenic/immunogenic in pathological conditions.

### BACKGROUND OF THE INVENTION

Type 1 diabetes is an autoimmune disease that results when a chronic inflammatory process of unknown origin destroys most of the insulin-producing β-cells in the pancreatic islets of Langerhans. Genetic susceptibility to diabetes is inherited and there is evidence that environmental co-factors strongly influence disease expression: <30% pairwise concordance in identical twins, 3.0% annual increase in global incidence since 1960 (Onkamo, P., et al,. (1999) Diabetologia 42, 1395-1403), wide geographic variation and results from numerous studies in animals showing environmental factors can modify the development of spontaneous autoimmune diabetes (Scott, F. W. (1996) Diabetes/Metabolism Reviews 12, 341-359; Akerblom, H. K., and M. Knip. (1998) Diabetes/Metabolism Reviews 14, 31-67). A major unresolved issue is the identification of the environmental factors that promote the development of type 1 diabetes. This task has proven difficult because of the multifactorial nature of the disease, difficulty in linking past exposures to development of diabetes, lack of knowledge of the environmental antigens, and the large number of predisposing genes in individuals at risk (Field, L. L. (2002) Diabetologia 45, 21-35).

The two most studied environmental factors are viruses and diet. Enteroviruses may be involved but as yet, a diabetes-inducing enterovirus has not been identified. Epidemiological evidence of infectious hotspots or traceable routes of infection is lacking and there are conflicting data with respect to the presence of candidate viruses in the pancreas or immune cells of diabetic patients (Juhela, S. et al. (2000) Diabetes 49, 1308-1313; Foulis, A. K:, et al.. (1997) Diabetologia 40, 53-61; Buesa-Gomez, J., et al, (1994) J Med Virol 42, 193-197). The highest incidence of spontaneous diabetes in Biobreeding (BB) rats and non obese diabetic (NOD) inice occurs when they are maintained in ultraclean conditions and gnotobiotic animals still develop diabetes. If animals that are maintained in strict viral-antibody-free conditions still develop diabetes, that leaves diet as the major environmental stimulus.

Although bovine proteins have been a central focus, a recent blinded, multi-center study demonstrated that a milk-free, wheat-based diet produced the highest diabetes frequency in diabetes-prone BB rats and NOD mice in three widely separate locations (Beales, P. et al., (2002) Diabetologia 45, 1240-1246), confirming numerous reports that the highest incidence of spontaneous diabetes occurs in animals fed mixed plant-based diets in which wheat is the major component. Defined diets in which wheat is the sole protein source are potent inducers of diabetes in BB rats (Scott, F. W. (1996) Diabetes/Metabolism Reviews 12, 341-359; Scott, F. W.et al. (1988) Adv Exp Med Biol 246, 277-285). In a different model of diabetes, the NOD mouse, wheat-based diets also resulted in high diabetes frequency (Coleman, D. L. et al., (1990) Diabetes 39,432-436; Karges, W., et al., (1997) Diabetes 46, 557-564; Hoorfar, J., et al., (1993) Br J Nutr 69, 597-607; Funda, D. P., et al., (1999) Diabetes Metab Res Rev 15, 323-327). In addition, an unusually high proportion of patients with type 1 diabetes (2-10%) have wheat gluten sensitive enteropathy (celiac disease, CD) (Lampasona, V., et al., (1999) Diabetologia 42, 1195-1198), a rate that is 10-33 times that in the normal population and about 1/3 of diabetes patients have antibodies against the CD autoantigen, tissue transglutaminase. Other reports indicate that increased peripheral blood T cell reactivity to wheat gluten was more frequent in newly diagnosed patients than in controls. These data are consistent with the involvement of dietary wheat proteins in diabetes pathogenesis.

Although considered to be a T cell mediated disease, studies of the prediction and pathogenesis of type 1 diabetes in humans rely heavily on serum autoantibodies as biomarkers of the destructive process. The humoral immune response to selected autoantigens correlates with histologic damage in the pancreas of newly diagnosed patients (Imagawa, A., et al., (2001) Diabetes 50, 1269-1273.).

The 64 kDa autoantigen originally reported in BB rat and human islets was identified in patients concordant for both the neurologic disease, Stiff-man syndrome and type 1 diabetes, as glutamic acid decarboxylase (GAD), a major autoantigen in type 1 diabetes (Baekkeskov, S., et al., (1990) Nature 347, 151-156). Despite continued progress, the antigens that initiate and maintain the process leading to β-cell destruction remain poorly understood.

Osman AA, Uhlig HH, Valdés I, Amin M, Méndez E, Mothes T. "Monoclonal antibody recognizing α potential coeliac toxic repetitive pentapeptide epitope in gliadins" in Eur. J. Gastroenterol. Hepatol. 2001; 13:1 189-1193, investigates a monoclonal antibody R5 that recognizes wheat gliadin, barley hordein and rye secalin equally. Both a phage-displayed heptapeptide library and overlapping peptides spanning the sequence of alpha- and gamma- type gliadins (pepscan) were screened for binding of R5. Both techniques yielded comparable pentapeptide consensus sequences (phage display QXPVV/FP; pepscan QQPFP). It is concluded that R5 seems to be a good candidate for the specifc detection of putative coeliac disease-active sequences in prolamins and thus represents a valuable tool for the quality control of gluten-free food.

DATABASE EMBL/GENBANK/DDBJ 6 August 2002 (2002-08-06), AL TENBACH, S. ET AL.: "WHE3558_A 12_B24ZS Wheat developing grains cDNA library Triticum aestivum" XP002470863 retrieved from EBI Database accession no. BQ804720, discloses a nucleotide sequence comprising a region encoding the epitope EEQLRELRRQ.

The development of autoimmune type 1 diabetes involves complex interactions among several genes and environmental agents. Human patients with type 1 diabetes show an unusually high frequency of wheat gluten-sensitive enteropathy, T cell response to wheat proteins is increased in some patients and high concentrations of wheat antibodies in blood have been reported. In both major models of spontaneous type 1 diabetes, the BB rat and NOD mouse, at least half of the cases are diet-related. In studies of BB rats fed defined semipurified diets, wheat gluten was a potent diabetes-inducing protein source. A major limitation in understanding how wheat or other dietary antigens affect type 1 diabetes has been the difficulty identifying specific diabetes-related dietary proteins.

There is a need in the art to identify proteins and nucleotide sequences encoding proteins which are diabetogenic in animals. Further, there is a need in the art to identify proteins, for example foodstuff proteins that are highly antigenic in overt diabetic animals. There is also a need in the art to develop screening processes to identify foodstuff proteins that are antigenic/immunogenic in subjects. Further, there is a need in the art to develop screening processes to identify subjects that may be at risk for developing a pathological condition due to consuming a foodstuff comprising an antigenic/immunogenic protein. There is also a need in the art to produce foods and foodstuffs in which one or more antigenic/immunogenic proteins are reduced or eliminated.

### SUMMARY OF THE INVENTION

The present invention relates to proteins which are antigenic/immunogenic in athological conditions.

According to the present invention there is provided an isolated amino acid sequence comprising a diabetogenic epitope, the diabetogenic epitope comprises the amino acid sequence EEQLRELRRQ (SEQ ID NO:1).

Also according to the present invention, there is provided a diabetogenic epitope as defined above comprising part of a larger peptide or protein that does not occur naturally in nature. In addition it is contemplated that the diabetogenic epitope is attached to a carrier protein, non-carrier protein, macromolecule or support. The support may comprise but is not limited to a bead, plate, dish, cover slip, slide, multiwell assay plate, or bio-assay chip.

The present invention also provides a nucleotide sequence encoding a diabetogenic epitope , the diabetogeic epitope is EEQLRELRRQ (SEQ ID NO:1).

Also provided by the present invention, there is provided nucleotide sequence complementary to a sequence encoding a diabetogenic epitope or a portion thereof.

The nucleotide sequence encoding a diabetogenic epitope, protein comprising a diabetogenic epitope or sequence complementary thereto may comprise part of a larger nucleotide sequence, for example a cloning vector or the like. The larger nucleotide sequence may comprise one or more regulatory sequences, for example, but not limited to express a nucleotide sequence encoding a diabetogenic epitope, protein comprising a diabetogenic epitope, or a sequence complementary thereto. The present invention further contemplates portions of nucleotide sequences encoding diabetogenic epitopes or proteins comprising diabetogenic epitopes or nucleotide sequences complimentary thereto, for example, but not limited to as probes. It is also possible that such probes may be labeled with any label known in the art.

The present invention also provides an isolated antibody capable of binding to a diabetogenic epitope comprising EEQLRELRRQ (SEQ ID NO:1).

Also provided by the present invention is an antibody as defined above which is a monoclonal antibody. In a further embodiment, the monoclonal antibody is an IgG antibody. The antibody may be produced in the serum of an animal, for example, but not limited to a diabetogenic animal, or an asymptomatic diabetic animal.

Also provided by the present invention is a kit comprising one or more of 1) a diabetogenic epitope, 2) a protein or peptide comprising a diabetogenic epitope, 3) a non-protein carrier or macromolecule comprising the diabetogenic epitope, 4) a support comprising the diabetogenic epitope, 5) a diabetogenic epitope attached to a non-covalent association agent 6) a nucleotide sequence encoding a diabetogenic epitope or peptide or protein comprising the diabetogenic epitope 7) a nucleotide sequence complementary to a nucleotide sequence encoding a diabetogenic epitope, 8) a nucleotide sequence complementary to a portion of a nucleotide sequence encoding a diabetogenic protein, or a combination thereof. In a preferred embodiment of the present invention, the diabetogenic epitope is from isoforms of gliadin proteins or Glb1. The diabetogenic epitope comprises EEQLRLELRRQ (SEQ ID NO:1).

The kit as defined above may further comprise one or more beads, plates, dishes, coverslips, slides, multi-well assay plates, bioassay chips, which may be attached or unattached to the diabetogenic epitope, protein or peptide comprising the diabetogenic epitope, nucleotide sequence encoding the diabetogenic epitope, sequence complementary thereto, or fragment thereof.

Further, it is contemplated that the kit as defined above may also comprise one or more primary antibodies capable of binding to the diabetogenic epitope, or protein comprising the diabetogenic epitope, one or more secondary antibodies that are capable of binding to the primary antibody, solutions, reagents, enzymes, or a combination thereof.

There is disclosed method of screening foodstuffs to identify proteins in the foodstuff which are antigenic/immunogenic in a subject, or group of subjects comprising a pathological condition, the method comprising the steps of:
a) processing the foodstuff to produce separated proteins, and;
b) screening the separated proteins from step a) with an antibody containing mixture derived from one or more subjects having the pathological condition to identify proteins that are antigenic/immunogenic in the subject and that are present in the foodstuff.

There is disclosed a method of screening foodstuffs to identify antigenic/immunogenic proteins common in at least two subjects, or groups of subjects wherein each subject or group of subjects comprise different pathological conditions, the method comprising the steps of
a) processing the foodstuff to produce separated proteins;
b) screening the separated proteins from step a) with a first antibody containing mixture derived from one or more subjects having a first pathological condition;
c) screening the separated proteins from step a) with a second antibody containing mixture derived from one or more subjects having a second pathological condition;
d) comparing proteins binding to the first antibody containing mixture with proteins binding to the second antibody mixture to identify proteins common in at least two subjects, or groups of subjects with different pathological conditions, the proteins also present in the foodstuff.

There is disclosed a foodstuff modified to reduce or eliminate one or more diabetogenic epitopes or proteins comprising diabetogenic epitopes. In an embodiment the foodstuff is modified to reduce or eliminate Glb1, isoforms of gliadin proteins, or a diabetogenic epitope thereof. For example, but not to be limiting in any manner, the foodstuff may be a genetically modified plant comprising a knockout of one or more diabetic epitopes or proteins comprising said one or more diabetic epitopes. In an embodiment, the genetically modified plant is a wheat plant.

Also contemplated by the present invention is a foodstuff which comprises an inhibitory RNA nucleotide sequence that reduces or eliminates the production of one or more proteins comprising one or more diabetogenic epitopes.

This summary of the invention does not necessarily describe all features of the invention.

### BRIEF DESCRIPTION OF THE DRAWINGS

These and other features of the invention will become more apparent from the following description in which reference is made to the appended drawings wherein:

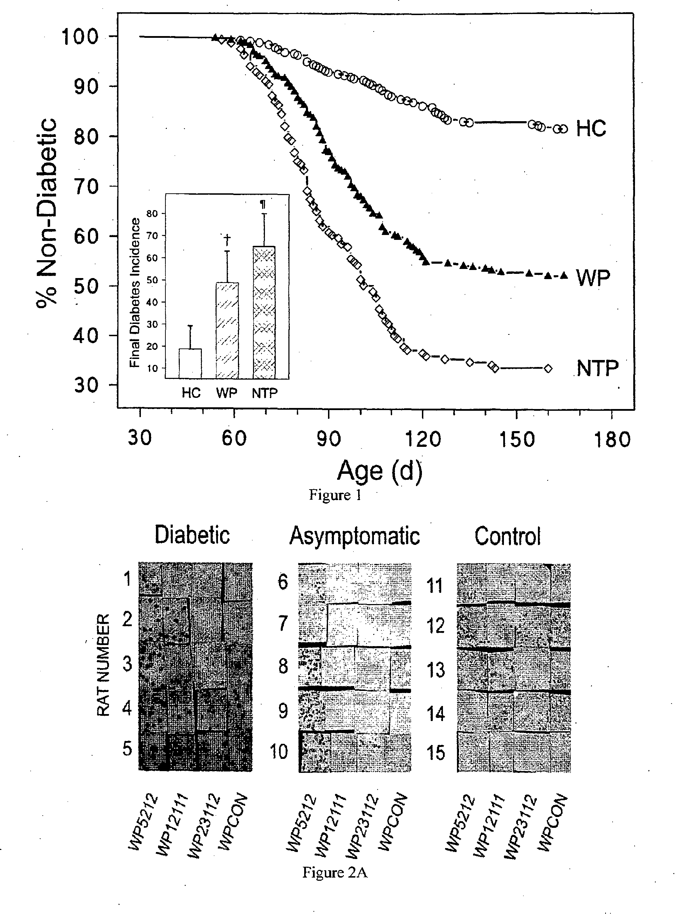

**Figure 1** shows the modification of diabetes development in diabetes-prone BB rats by wheat-based diets. Survival curves and final diabetes incidence (inset) in BBdp rats fed from weaning a mixed, cereal-based, mainly wheat-based, NTP-2000 (National Toxicology Program, NTP) diet, or two semipurified, isocaloric, isonitrogenous diets in which the sole amino acid source was either hydrolyzed casein (HC) or wheat proteins (WP) plus supplemental sulphur amino acids. Animals fed the NTP-2000 diet had the highest incidence, 65.3 ± 14.9 % (n=6 experiments, total of 169 rats, Figure 1; ¶, denotes p< 10⁻⁶ vs. HC). There were more cases of diabetes in BBdp rats fed WP diets (n=12 experiments, total of 282 rats, 50.6 ± 11.1%) than those fed a protective HC diet (n=14 experiments, total of 322 rats, 18.8 ± 10.6%, 14 experiments; ⊥ denotes p= 10⁻⁵).

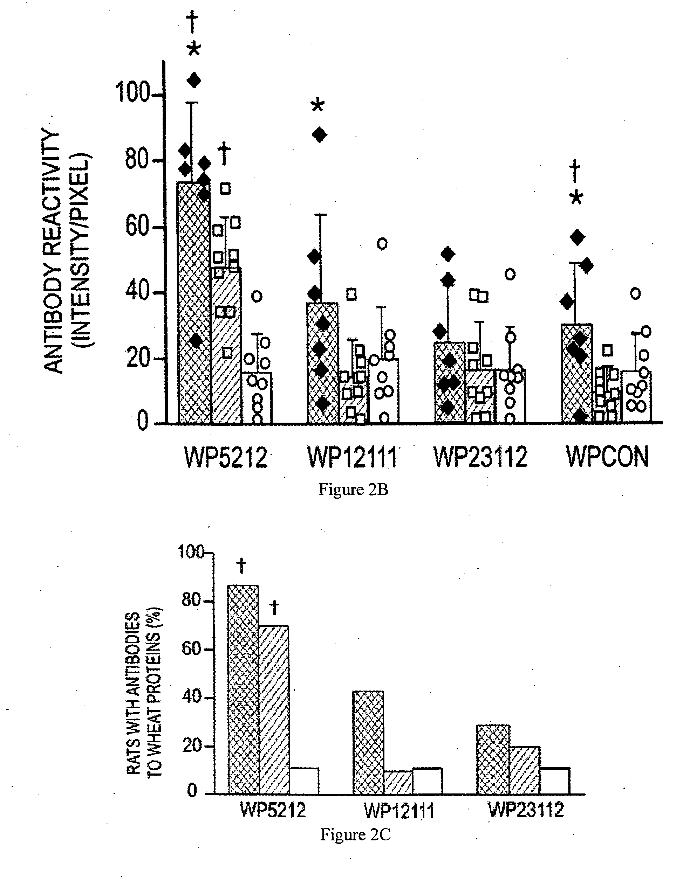

**Figure 2** shows examples of (A) plaque lifts of clones screened with serum from diabetic, asymptomatic or control rats; (B) antibody reactivity to three clones and (C) frequency of antibody reactivity to the wheat proteins. **Figure 2A** shows plaque lifts of clones WP5212, WP12111, WP23112 and WPCON screened with serum from five diabetic, asymptomatic or control rats. **Figure 2B** shows mean antibody reactivity (intensity/pixel) ± SD to the recombinant wheat proteins screened with diabetic (cross-hatch), asymptomatic (hatched) or control (open) BB rats are shown. Individual values for diabetic (diamonds), asymptomatic (squares) or control (circles) rats are shown. **Figure 2C** shows the frequency of diabetic (cross-hatched), asymptomatic (hatched), and control (open) BB rats with positive antibody reactivity to the wheat proteins is shown. A positive antibody level was defined as an antibody reactivity greater than the mean intensity of WPCON screened with control rat serum plus two SD. (ANOVA/LSD; † indicates significant difference vs control rats, p≤0.02; * indicates significant vs asymptomatic rats, p≤0.02).

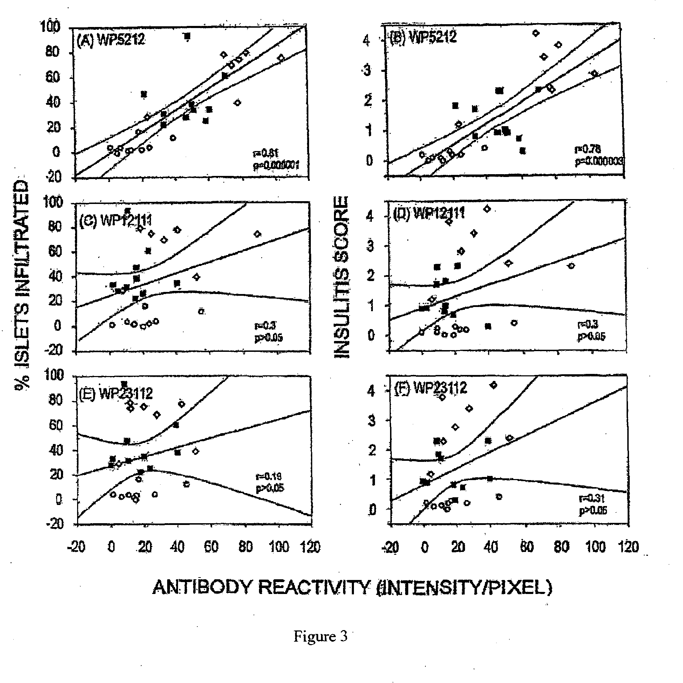

**Figure 3** shows antibody reactivity to the Glb1. clone is strongly asociated with pancreatic inflammation or insulitis. The correlation between the percent of islets infiltrated (left column) or insulitis score (right column) and antibody reactivity (mean intensity/pixel) to three recombinant wheat proteins in diabetic (diamonds), asymptomatic (squares) or control (circles) rats are shown. The Pearson Product-Moment correlation r and p values are indicated.

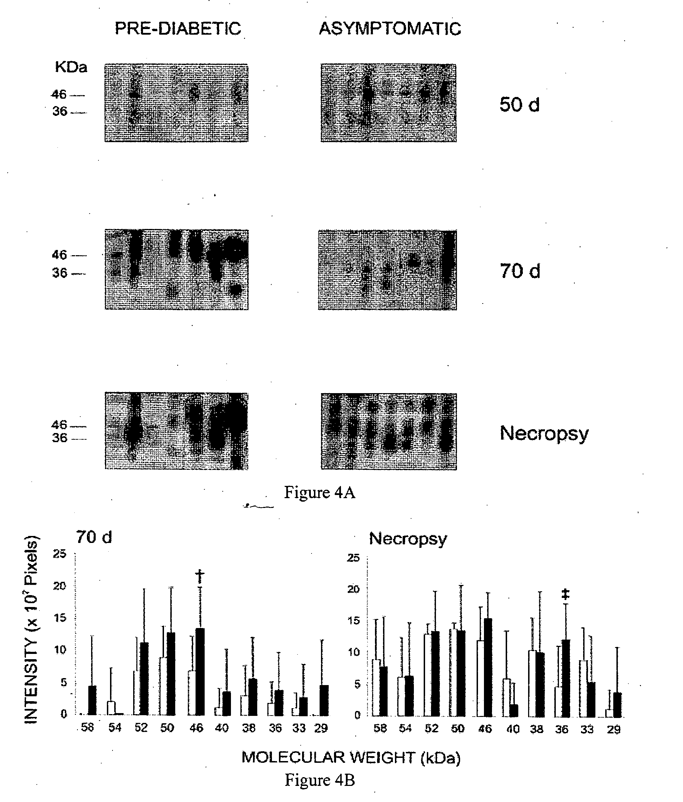

**Figure 4** shows 1D Western analysis of wheat proteins probed with serum collected prospectively from BB rats at different risk of developing diabetes. 1D Western blots of wheat proteins probed with serum from prediabetic or asymptomatic BB rats at 50 d, 70 d and necropsy are shown in **Figure 4A**. The mean intensity ∀ SD of each wheat protein band is shown for the prediabetic period (70 d) or at necropsy for asymptomatic (open bars) or diabetic (filled bars) in **Figure 4B**; (ANOVA/LSD; †, p=0.02; ‡, p=0.006).

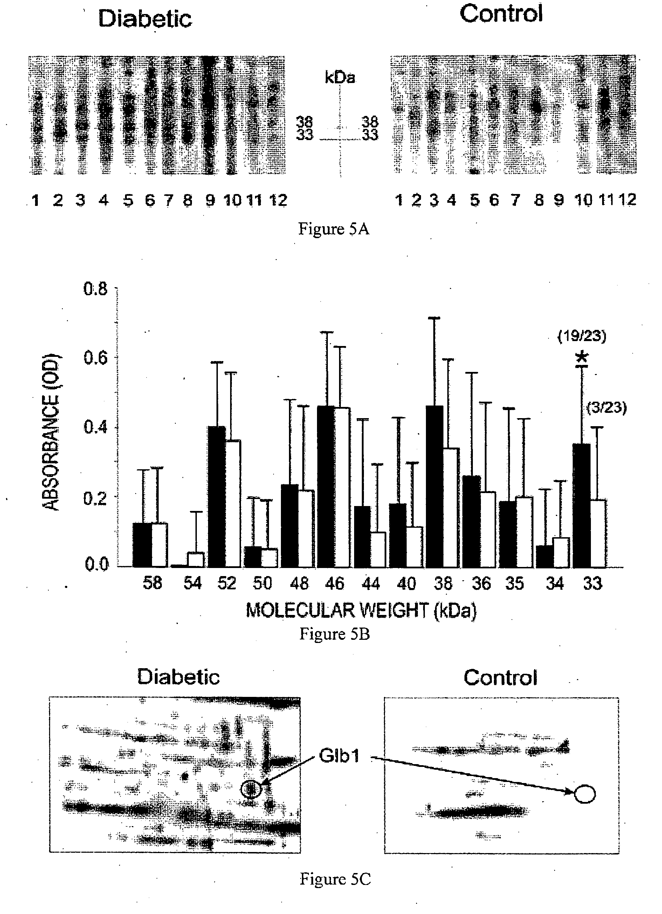

. **Figure 5** shows 1D and 2D Western analysis of antibody binding to wheat proteins in patients and HLA-DQ matched controls. **Figure 5A** shows 1D Western blots of wheat proteins probed with serum samples from diabetic children and control children without diabetes. **Figure 5B** shows the mean absorbance + SD of (each) wheat protein band probed with serum from diabetic children (filled bars) and HLA-DQ-matched controls (open bars) (ANOVA/LSD; * indicates p=0.005). **Figure 5C** shows 2D Western blot of wheat proteins probed with pooled serum samples from newly diagnosed diabetic children (left) or control children (right). Wheat storage globulin, Glb1, was bound by antibodies in serum from children with diabetes but there was no binding using serum from non-diabetic controls.

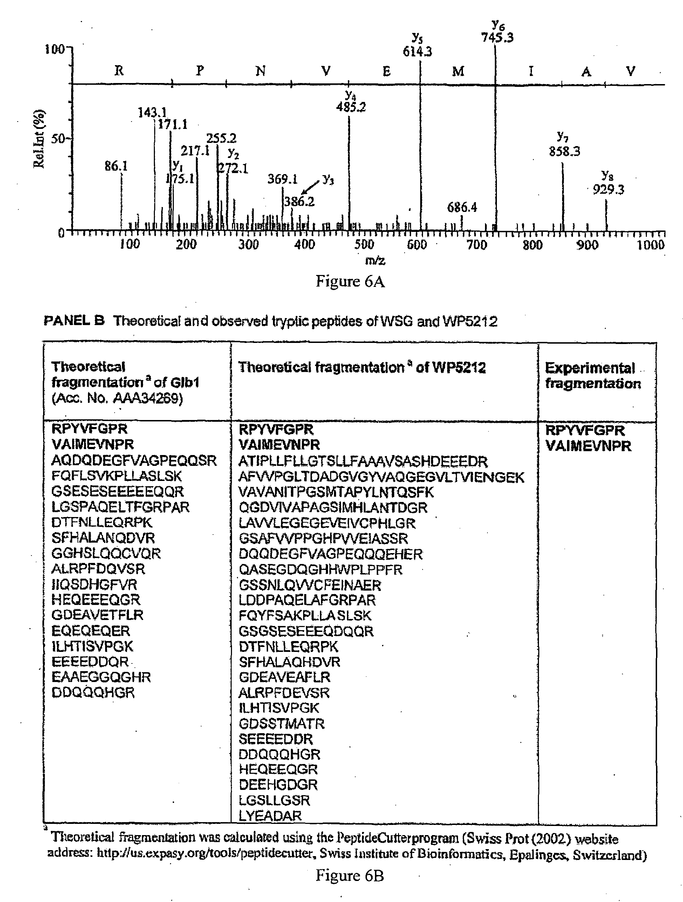

**Figure 6** shows identification of wheat storage globulin, Glb1, by in-gel tryptic digestion and capLC-MS/MS analysis.

**Figure 6A** shows the MS/MS spectrum of the doubly protonated ion (MH₂²⁺) at m/z 514.8 corresponding to the Glb1 tryptic peptide, VAIMEVNPR (SEQ ID NO:2). The sequence of this peptide can be determined from the y-ion series (i.e., fragment ions that originate from the C-terminus of the peptide) as is indicated on the spectrum. **Figure 6B** shows theoretical and observed tryptic peptides of WSG and WP5212. In the first column, the sequence identifiers from top to bottom are SEQ ID NOs:14-31. In the second column, the sequence identifiers from top to bottom are SEQ ID NOs: 14-15, 32-43, 20, 44-46, 28, 47-48, 31, and 49-52. In the third column, the sequence identifiers from top to bottom are SEQ ID NOs:14-15.

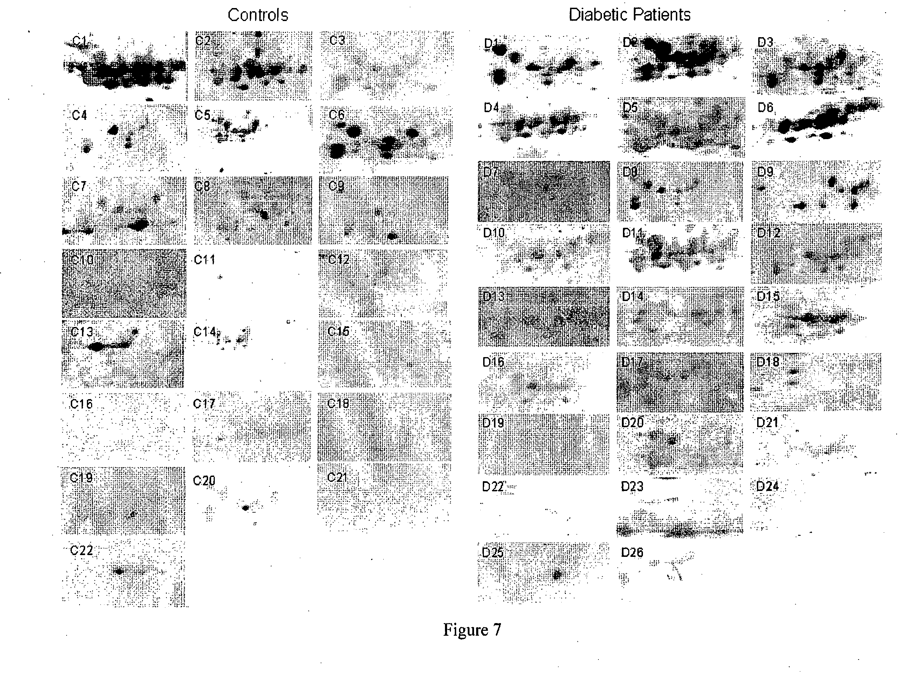

**Figure 7** shows increased IgG reactivity to wheat proteins mainly in adult T1D patients. 2D Western blots of patients with type 1 diabetes (average age 24.6+/- 6.8 y, n=7) and patients with type 1 diabetes (25.4+/- 8.1y, n=26) are shown. WG extract was resolved using 2D-electrophoresis and blotted onto nitrocellulose. Proteins were probed with serum from patients and IgG binding was detected using enhanced chemiluminescence. With the exception of patients D8 and D9 who are children 8 years old, both groups were closely matched for age and sex.

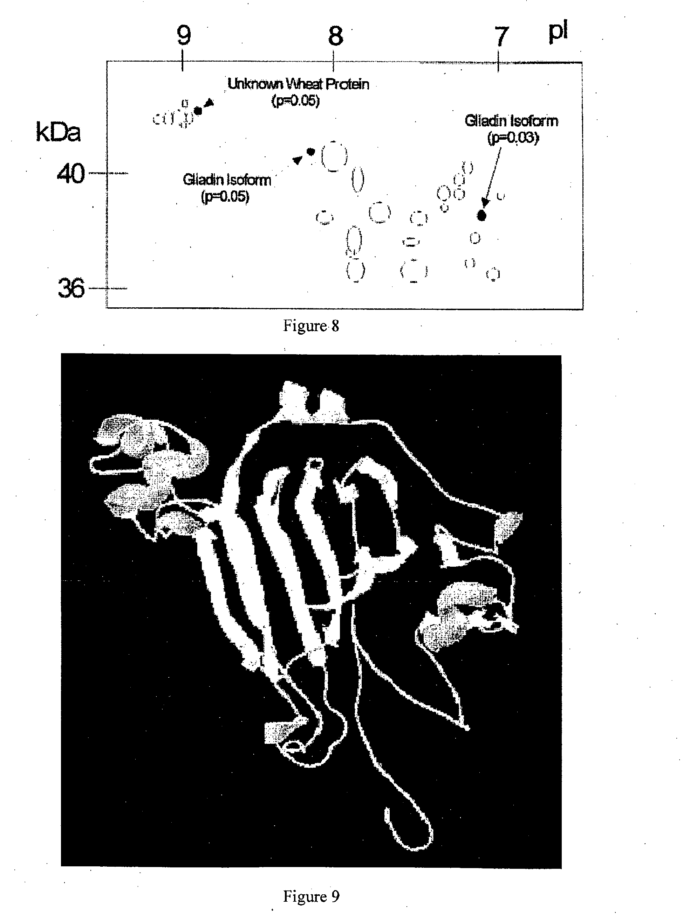

**Figure 8** shows results indicating increased reactivity to wheat proteins in T1D patients. The filled spots represent proteins that were more frequently antigenic in T1D patients compared with controls (p≤0.05). These were gliadin isoforms and an unknown wheat protein.

**Figure 9** shows the predicted three dimensional structure of WP5212.

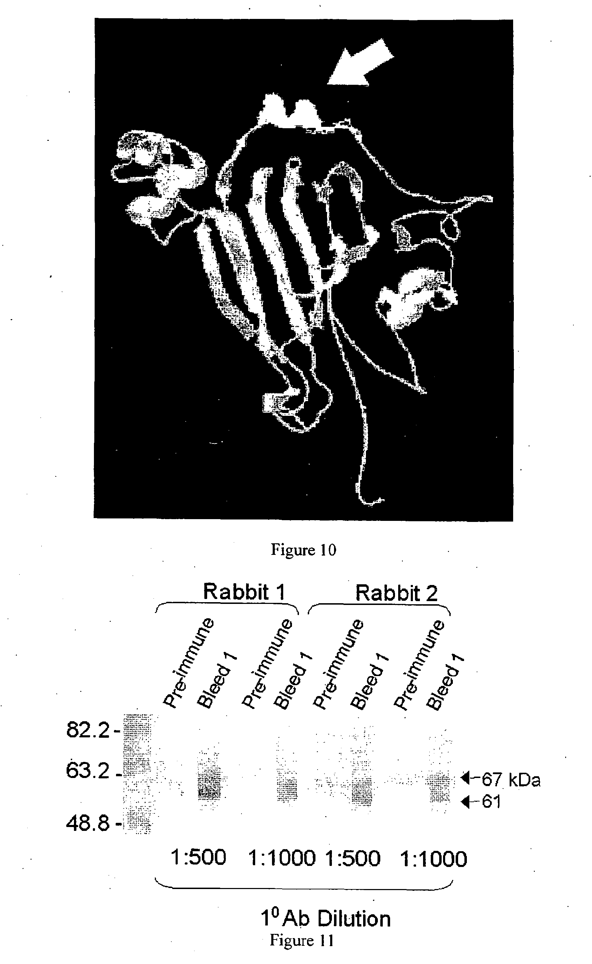

**Figure 10** shows a potential three dimensional structure of the antigenic epitope of WP5212 as shown by the arrow.

**Figure 11** shows results confirming WP5212 expression in Sf21 insect cells by probing Western blots with polyclonal WP5212-specific antibodies. 1D Western blots were probed with serum diluted 1:500 or 1:1000 in blocking buffer from two rabbits immunized with WP5212 specific peptides. Pre-immune serum does not bind the 65 kDa WP5212 protein. WP5212 is expressed as a doublet in Sf21 insect cells and appears as 61 and 67 kDa isoforms.

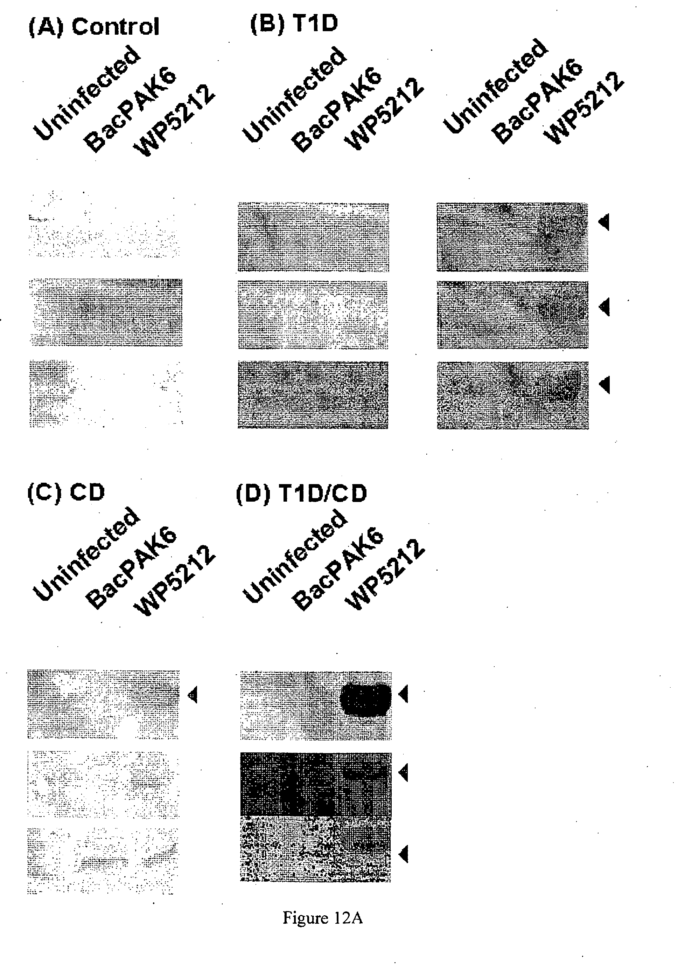

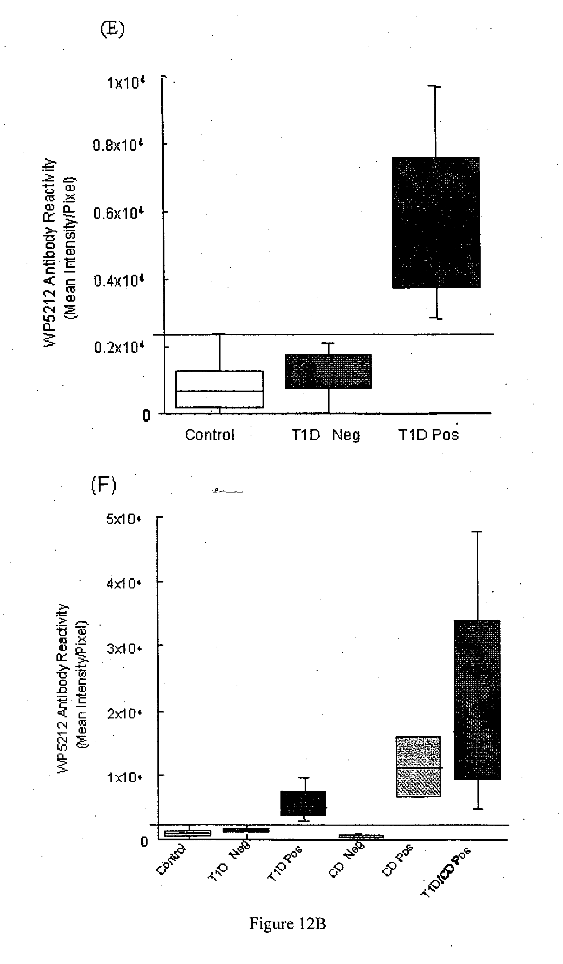

**Figure 12** shows results indicating T1D and T1D/CD patients have increased WP5212 antibody reactivity in comparison to control subjects. Panels A to D, examples of 1D SDS-PAGE Western blots of uninfected, parental BacPAK6 infected or recombinant WP5212 baculovirus infected insect cell lysate (10 µg/lane) were screened with serum antibodies from healthy control subjects (A), patients with T1D (B), CD (C) or both T1D and CD (D). Arrowhead markers indicate WP5212 antibody positive individuals. Panels E and F, densitometric analysis was performed to determine WP5212 antibody reactivity of control subjects (E and F), patients with T1D (E and F) or CD (F), or with both T1D and CD (F). Subjects with antibody reactivity values greater than the mean + 2SD of the control group (horizontal line) were deemed positive. *, p ≤ 0.01 vs T1D patients; #, p ≤ 0.05 vs T1D/CD patients.

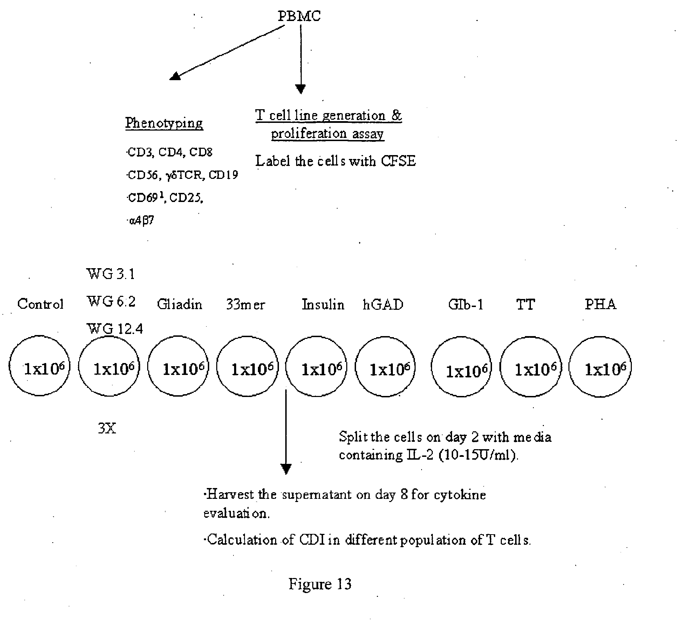

**Figure 13** shows a flow chart depiction of the antigen-specific CD3+ T cell proliferation of PBMC by CFSE assay.

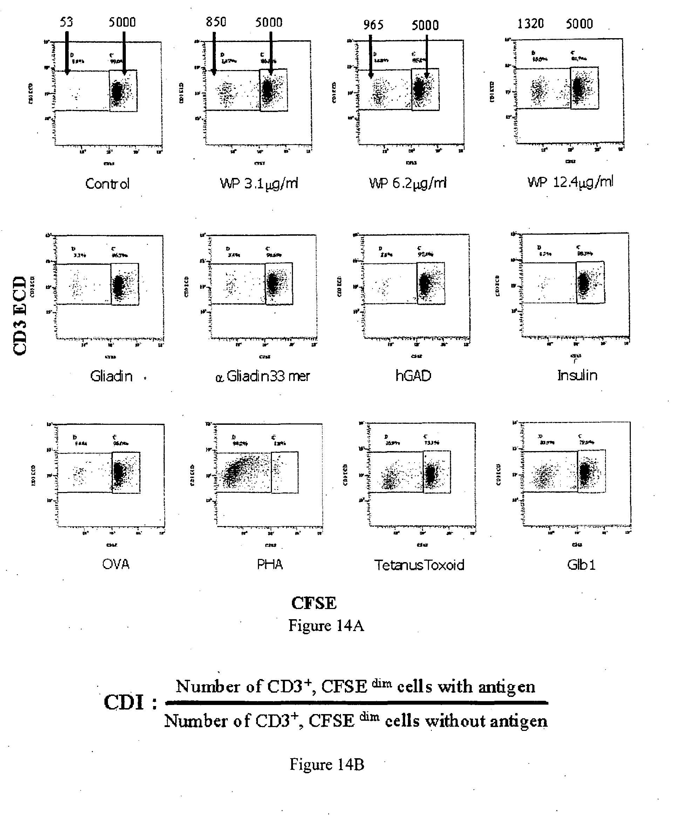

**Figure 14** shows FACS plots of CFSE-labeled CD3 ECD-stained PBMC after 8 d of culture +/- antigens (**Figure 14A**). The number of CFSEdim events was the number corresponding to 5000 CD3+ CFSEbright events. The CDI was calculated based on a fixed number of 5000 CD3+ CFSEbright cells using the formula shown (**Figure 14B**).

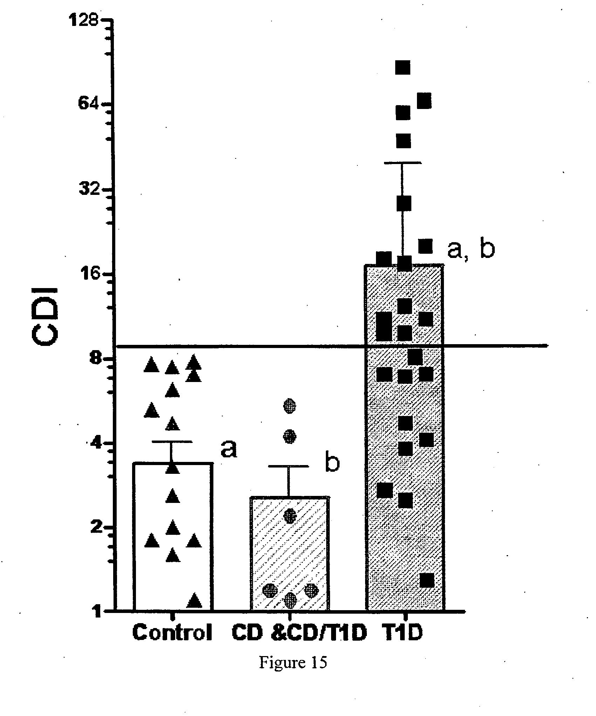

**Figure 15** shows graphically the response to WP in individual donors evaluated with CFSE proliferation assay. Distribution of CDI to wheat protein in patients with T1D (n=26), CD or CD/T1D (n=6) and healthy control subjects (n=19). CDI value of the mean + 2 SD (8.77) was used as a cutoff for positive proliferation response. A positive response to WP was found in 13 patients with T1D (50%; p<0.01 vs. CD & CD/T1D patients and controls).

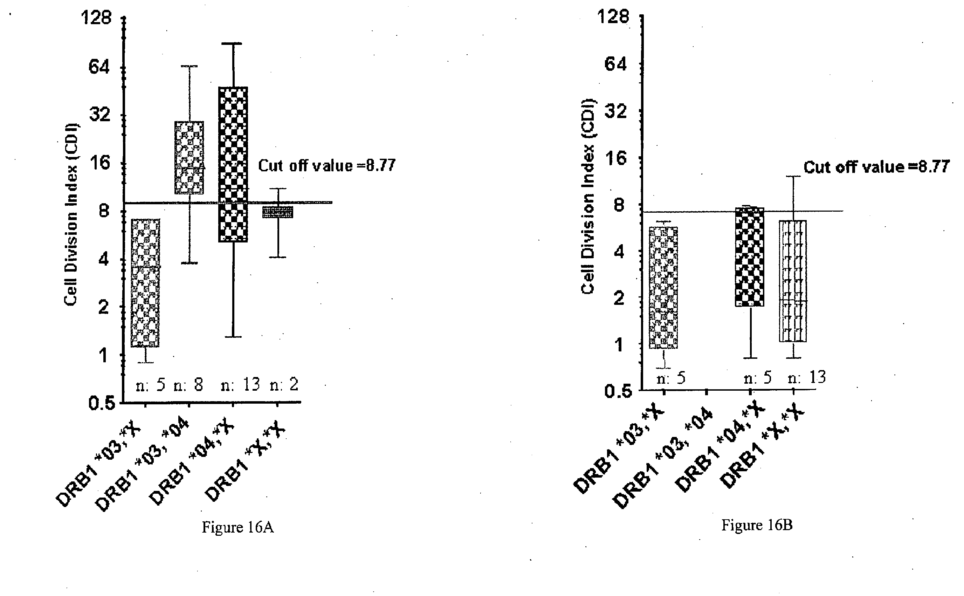

**Figure 16** shows graphically the relation between CDI to wheat protein and HLA DRB1 diabetes risk alleles in T1D patients (A), and in control subjects (B). Values above the horizontal line (CDI + 2 SD) were designated as responders. X stands for alleles other than DRB1 *03 or *04. These data show that the positive response to WP in T1D is associated with the high risk diabetes gene, HLA DR4,

### DETAILED DESCRIPTION

The following description is of a preferred embodiment, which is not meant to be limiting in any manner.

### Peptide Sequences

According to an embodiment of the present invention, there is provided a peptide or protein sequence comprising at least one diabetogenic epitope.

By the term "diabetogenic epitope" it is meant a sequence of amino acids which is capable of being bound by an antibody produced by a subject, for example, but not limited to a human subject, the antibody involved in an immune reaction associated with diabetes or diabetes pathogenesis. The epitope may comprise a linear sequence of amino acids which is recognized by the antibody, or the epitope may adopt a higher ordered structure, for example, a three dimensional structure as is known in the art, and the antibody may bind to the three dimensional structure of the epitope.

In an embodiment, which is not meant to be considered limiting in any manner, the peptide sequence comprises a diabetogenic epitope from isoforms of gliadin peptides, for example, but not limited to α, β-, γ- and ω-gliadins. Alternatively, the diabetogenic epitope may be from Glb1. In separate embodiments, which are not meant to be limiting, the diabetogenic epitope may be from α/β gliadin AII precursor or α/β gliadin MM1 precursor. The nucleotide sequence of WP5212 and amino acid sequences of these proteins are shown in Tables 1 and 2. In a further embodiment of the present invention the diabetogenic epitope is EEQLRELRRQ (SEQ ID NO:1) (shown from N-terminus to C-terminus) from Glb1.

| Table 1: Wheat gene sequences | | |
|---|---|---|
| Gene Name and ID No. | Database | Nucleotide Sequence |
| WP5212 (Homologue to globulin Beg1 precursor TC103916¹ ) | TIGR Wheat Gene Index | |

| Table 2: Selected wheat protein sequences | | |
|---|---|---|
| Protein Name and ID No. | Database | Amino Acid Sequence |
| WP5212² | n/a | |
| | | |
| alpha/betagliadin A-II precursor P04722 | NCBI | |
| alpha/betagliadin MM1 precursor P18573 | NCBI | |
| gammagliadin [Triticum aestivum AAK84779.1 | NCBI | |
| (Gliadin Isoform, p=0.05) | | |
| gammagliadin [Triticum aestivum] AAK84777.1 | NCBI | |
| (Gliadin isoform p=0.03) | | |
| ¹Recently a new EST was submitted to the TIGR Wheat Gene Index that matched exactly the sequence for WP5212, referred to as Glb.1 | | |
| ²The expected translation of the open reading frame of WP5212. | | |

It is also contemplated that the diabetogenic epitope may comprise part of a larger peptide or protein. For example, but not wishing to be limiting in any manner, one or more amino acids may be attached via one or more peptide bonds to the diabetogenic epitope at the N-terminal amino acid, the C-terminal amino acid or both. Further, the diabetogenic epitope may be attached covalently or non-covalently to a carrier protein, for example, but not limited to serum albumin such as BSA, KLH or other suitable carrier. It is also contemplated that the diabetogenic epitope may be attached in series to form a homopolymer for example, but not limited to EEQLRELRRQEEQLRELRRQ (SEQ ID NO:9). In the event that the diabetogenic epitope is attached to a carrier protein or other peptide or amino acid sequence, preferably it is attached via a covalent bond, for example a peptide bond or other covalent bond.

It is also contemplated that the diabetogenic epitope, peptide comprising the diabetogenic epitope, or carrier protein attached thereto may comprise a purification tag, for example, but not limited to a hexahistidine tag to facilitate purification, an amino acid spacer sequence for example, but not limited to reduce steric hindrance during binding of the diabetogenic epitope to an antibody or the like, a non-covalent association agent such as, but not limited to biotin to promote association between the diabetogenic epitope and avidin or avidin-like molecule, for example, but not limited to streptavidin.

The diabetogenic epitope also may be covalently attached or non-covalently associated with a non-protein carrier or macromolecule for example, but not limited to polyethylene glycol, dextran or the like, or it may be covalently attached or non-covalently associated with a support for example, but not limited to a bead, plate, dish, cover slip, slide, multiwell assay plate, bio-assay chip, and the like manufactured from any suitable material known in the art. Representative examples of such materials may include, but are not limited to glass, and plastic for example, but not limited to polystyrene, polypropylene, and the like. A variety of methods exist in the art to attach, couple, bind or associate the diabetogenic epitope with a non-protein carrier or support, and any such method is meant to be encompassed within the scope of the present invention.

As indicated previously, the diabetogenic epitope may form part of a larger protein or macromolecule. Preferably, the larger protein or macromolecule does not naturally occur in nature. Also, it is preferred that the diabetogenic epitope is sterically unhindered such that it is capable of being bound by an antibody. More preferably, the diabetogenic epitope forms part of a surface region such that an antibody specific for such a sequence is capable of binding to it under about normal physiological conditions, for example conditions similar to those in which an antibody binds to an antigen in an organism from which the antibody is naturally produced.

The diabetogenic epitope alone or attached to a carrier protein, non-protein carrier, macromolecule, support or combination thereof may be prepared according to a variety of methods known in the art. For example, proteins or peptides comprising the diabetogenic epitope may be prepared using standard techniques in molecular biology, for example by 1) transforming a suitable cell with an expression vector comprising a nucleotide sequence encoding the diabetogenic epitope or a peptide or protein comprising the diabetogenic epitope, and 2) expressing the protein or peptide in the cell. Alternatively, a diabetogenic epitope, peptide comprising the diabetogenic epitope, or protein comprising the diabetogenic epitope may be prepared by peptide chemistry for example, but not limited to solid phase or solution phase peptide synthesis. Preferably the diabetogenic epitope, or protein comprising the diabetogenic epitope is relatively short, for example, but not limited to less than about 50 amino acids in length, more preferably less than about 30 amino acids in length, and still more preferably less than about 20 amino acids in length. Macromolecules, non-protein carriers, supports and the like may be prepared by standard techniques known in the art and any method known in the art to attach a diabetogenic epitope or protein comprising a diabetogenic epitope thereto may be employed in the present invention. It is also contemplated that a combination approach using molecular biology and other techniques may be employed.

The amino acid sequence of the diabetogenic epitope, or protein comprising the diabetogenic epitope may be used to screen for animals or humans that develop one or more antibodies that bind to the diabetogenic epitope. In this manner it may be possible to screen for animals that have diabetes or that are at risk or predisposed to developing diabetes. For example, the diabetogenic epitope, or protein comprising the diabetogenic epitope may be contacted with immune serum from an animal. Binding of an antibody in the sera of an animal to the diabetogenic epitope is indicative that the animal may be at risk for developing type 1 diabetes.

### Nucleotide Sequences

There is contemplated a nucleotide sequence encoding a diabetogenic epitope, or a portion thereof, the diabetogenic epitope derived from a protein selected from the group consisting of, but not limited to, isoforms of gliadin proteins and Glb1. In an embodiment of the present invention, which is not meant to be limiting in any manner, the nucleotide sequence encodes an epitope defined by the amino acid sequence EEQLRELRRQ (SEQ ID NO:1). As will be evident to a person of skill in the art, a particular protein sequence may be encoded by numerous DNA sequences due to the degeneracy of the genetic code and thus multiple nucleotide sequences may encode the same diabetogenic epitope. All such nucleotide sequences are meant to be encompassed by the present invention.

In an alternate embodiment of the present invention, the nucleotide sequence may be complementary to a sequence encoding a diabetogenic epitope or a portion thereof. The complementary nucleotide sequence may be employed as a probe to identify sequences of DNA in organisms such as, but not limited to plants which may encode proteins comprising diabetogenic epitopes. Alternatively, but without wishing to be limiting, the complementary nucleotide sequence may be employed as a primer, for example in PCR reactions and the like. In an embodiment of the present invention, the complementary nucleotide sequence comprises greater than about 10 nucleotides, preferably greater than about 17 nucleotides, more preferably greater than about 21 nucleotides. In still another embodiment, the complementary nucleotide sequence may be longer, for example, but not limited to in the range of about 50 to about 150 nucleotides.

The nucleotide sequence of the present invention also encompasses nucleotide sequences encoding diabetogenic epitopes such as but not limited to EEQLRELRRQ (SEQ ID NO:1) and complementary nucleotide sequences or portions thereof which hybridize under stringent hybridization conditions (see Maniatis et al., in Molecular Cloning (A Laboratory Manual), Cold Spring Harbor Laboratory (1982) p 387 to 389). An example of one such stringent hybridization condition may be hybridization at 4XSSC at 65°C, followed by washing in 0.1XSSC at 65°C for an hour. Alternatively an exemplary stringent hybridization condition could be in 50% formamide, 4XSSC at 42°C.

It is also contemplated that the nucleotide sequence encoding a diabetogenic epitope may form part of a larger nucleotide sequence, for example, but not limited to a vector that further comprises one or more regulatory sequences including, but not limited to promoter elements, basal (core) promoter elements, elements that are inducible in response to an external stimulus, elements that mediate promoter activity such as negative regulatory sequences or transcriptional enhancers. Regulatory sequences may also comprise elements that are active following transcription, for example, regulatory sequences that modulate gene expression such as translational and transcriptional enhancers, translational and transcriptional repressors, upstream activating sequences, and mRNA instability determinants. Several of these latter elements may be located proximal to the coding region. In the context of this disclosure, the regulatory sequence typically refers to a sequence of DNA, usually, but not always, upstream (5') to the coding sequence of a structural gene, which controls the expression of the coding region by providing the recognition for RNA polymerase and/or other factors required for transcription to start at a particular site. However, it is to be understood that other nucleotide sequences, located within introns, or 3' of the sequence may also contribute to the regulation of expression of a coding region of interest. An example of a regulatory sequence that provides for the recognition for RNA polymerase or other transcriptional factors to ensure initiation at a particular site is a promoter sequence. A promoter sequence comprises a basal promoter sequence, responsible for the initiation of transcription, as well as other regulatory sequences (as listed above) that modify gene expression.

There are also several types of regulatory sequences, including those that are developmentally regulated, inducible and constitutive. A regulatory sequence that is developmentally regulated, or controls the differential expression of a gene under its control, is activated within certain organs or tissues of an organ at specific times during the development of that organ or tissue. However, some regulatory sequences that are developmentally regulated may preferentially be active within certain organs or tissues at specific developmental stages, they may also be active in a developmentally regulated manner, or at a basal level in other organs or tissues within the plant as well.

An inducible regulatory sequence is one that is capable of directly or indirectly activating transcription of one or more DNA sequences or genes in response to an inducer. In the absence of an inducer the DNA sequences or genes will not be transcribed. Typically the protein factor, that binds specifically to an inducible sequence to activate transcription, may be present in an inactive form which is then directly or indirectly converted to the active form by the inducer. However, the protein factor may also be absent. The inducer can be a chemical agent such as a protein, metabolite, growth regulator, herbicide or phenolic compound or a physiological stress imposed directly by heat, cold, salt, or toxic elements or indirectly through the action of a pathogen or disease agent such as a virus. Any regulatory sequence known in the art may comprise part of the nucleotide sequence encoding a diabetogenic epitope as described herein. Further, as it is equally contemplated that the nucleotide sequence encoding a diabetogenic epitope may be produced by a variety of cells, for example, but not limited to bacterial cells, yeast cells, insect cells, or mammalian cells, the present invention contemplates the use of any regulatory element known in the art, for example, but not limited to promoter, terminator, upstream activation sequence, enhancer, origin of replication, or any combination thereof in the nucleotide sequence of the present invention.

The nucleotide sequence encoding a diabetogenic epitope may also comprise a label for example, but not limited to a radiolabel, heavy metal particle, for example, but not limited to gold, silver or the like, a fluorescent group, or the like to facilitate identification of the sequence during use. Any label known in the art is meant to be encompassed by the present invention.

Nucleotide sequences encoding a diabetogenic epitope or protein may be employed for mass production of such proteins or epitope sequences. Alternatively, the nucleotide sequences complementary thereto may be employed in a method to identify DNA in organisms that may be translated to produce potentially diabetogenic proteins. For example, but not wishing to be limiting in any manner, a food plant, such as, but not limited to wheat may be screened to determine whether the plant may comprise DNA that produces a diabetogenic protein.

### Antibodies

The present invention also provides an isolated antibody capable of binding to Glb1 or isoforms of gliadin proteins. Preferably, the antibody binds to a diabetogenic epitope of isoforms of gliadin proteins or Glb1, for example, but not limited to the amino acid sequence EEQLRELRRQ (SEQ ID NO:1). In a preferred embodiment, the antibody is a monoclonal antibody, more preferably an Ig-G monoclonal antibody. The antibody may be derived from any antibody producing species, for example, but not limited to mouse, rat, human, goat, rabbit, etc. Further, the antibody may be produced by immunizing an animal, with the diabetogenic epitope, peptide or protein comprising the diabetogenic epitope, or non-protein carrier comprising a diabetogenic epitope. The production of antibodies may be performed by a person of skill using any one of a variety of methods known in the art, for example, but not limited to Harlow and Lane, "Antibodies a laboratory manual", (Cold Spring Harbor Laboratory Press, Cold Spring Harbor, 1988). Alternatively, the isolated antibody may be obtained from the serum of an animal that comprises one or more antibodies specific for a diabetogenic epitope. A variety of methods are known in the art for purifying antibodies and any such method may be employed in the present invention.

In an embodiment, which is not meant to be limiting in any manner, there is provided a method of producing one or more antibodies against one or more diabetogeic epitopes of one or more proteins in an animal comprising the steps of injecting an immunogenic composition comprising one or more diabetogenic epitopes into the animal and isolating one or more antibodies from the animal. The "injecting" may comprise one or several injections spaced over appropriate intervals as would be known to someone of skill in the art. Further, the immunogenic composition may comprise an adjuvant to augment the production of an immune response in an animal.

### Kits

There is contemplated a kit comprising one or more of: 1) a diabetogenic epitope, 2) a protein or peptide comprising a diabetogenic epitope, 3) a non-protein carrier or macromolecule comprising the diabetogenic epitope, 4) a support comprising the diabetogenic epitope, 5) a diabetogenic epitope attached to a non-covalent association agent, 6) a nucleotide sequence encoding a diabetogenic epitope or peptide or protein comprising the diabetogenic epitope, 7) a nucleotide sequence complementary to a nucleotide sequence encoding a diabetogenic epitope, 8) a nucleotide sequence complementary to a portion of a nucleotide sequence encoding a diabetogenic protein, or any combination thereof.

In an embodiment of the present invention which is not meant to be limiting in any manner, the diabetogenic epitope is from isoforms of gliadin proteins or Glb1. In a specific embodiment of the present invention the diabetogenic epitope is EEQLRELRRQ (SEQ ID NO:1) from Glb1. In an alternate embodiment, which is not meant to be limiting in any manner, the diabetogenic epitope may be from one or more isoforms of gliadin proteins and Glb1.

It is also contemplated that the kit may comprise a protein or peptide comprising a diabetogenic epitope, for example, but not limited to isoforms of gliadin proteins or Glb-1. The kit may also comprise combinations of these proteins.

As disclosed earlier, any nucleotide sequence defined above may be part of a larger nucleotide sequence, for example, but not limited to cloning vector or the like. The larger nucleotide sequence, for example, cloning vector or the like may comprise additional nucleotide sequences for example, but not limited to regulatory sequences. Also the nucleotide sequence as disclosed above may comprise a label, for example, but not limited to P-32 label to aid in identification of the sequence.

The kits of the present invention may also comprise one or more beads, plates, dishes, coverslips, slides, multi-well assay plates, bioassay chips, which may be attached or unattached to the diabetogenic epitope, protein or peptide comprising the diabetogenic epitope, nucleotide sequence encoding the diabetogenic epitope, sequence complementary thereto, or fragment thereof.

The present invention also contemplates a panel of proteins such as but not limited to Glb-1 and gliadin isoforms that can be employed as screening agents. Without wishing to be limiting, such a panel of proteins may be attached to a support as described herein.

The kits of the present invention may further comprise other components, for example, but not limited to one or more primary antibodies capable of binding to the diabetogenic epitope, or protein comprising the diabetogenic epitope. Preferably the primary antibody binds to the diabetogenic epitope. The kits may also comprise a secondary antibody which is capable of binding to the primary antibody. It is also possible that the kit may comprise a tertiary antibody, (or higher order antibodies) which is capable of binding the secondary antibody. The secondary, tertiary or higher order antibodies may be labeled for example, but not limited to provide a signal to aid in identification of binding.

The kit may also comprise one or more solutions, reagents, enzymes or combinations thereof. For example, the kit may comprise protein or nucleotide sequence binding solutions, washing solutions, blocking solutions, substrate solution, for example but not limited to enzyme substrate solutions and solutions permitting chemiluminescence. The kit may also comprise assay instructions for example, but not limited to any method as disclosed herein.

The kit comprising one or more diabetogenic proteins, peptides or fragments thereof may be used to identify subjects that comprise one or more antibodies against a diabetogenic epitope or protein comprising a diabetogenic epitope. For example, the one or more diabetogenic proteins, peptides or fragments thereof may be contacted with immune serum from an animal or human. Further, the kits may be employed in screens to identify animals or humans at risk or predisposed to developing diabetes. Binding of an antibody in the sera of an animal or human to a diabetogenic protein, peptide, more preferably a diabetogenic epitope in the protein or peptide may be indicative that the animal or human is prone to diabetes. The kits also may be employed to identify foodstuffs which comprise proteins that comprise diabetogenic epitopes, or nucleotide sequences that may encode proteins comprising diabetogenic epitopes.

### Methods

There is disclosed a method of screening foodstuffs to identify proteins in the foodstuff which are antigenic/immunogenic in a subject, or groups of subjects comprising a pathological condition, the method comprising the steps of
a) processing the foodstuff to produce separated proteins, and;
b) screening the separated proteins from step a) with an antibody containing mixture derived from one or more subjects having the pathological condition to
identify proteins that are antigenic/immunogenic in the subject and that are present in the foodstuff.

By the term "foodstuffs" it is meant any food component that may be consumed by a subject. The foodstuffs may include foods such as food plants that are cultivated or occur naturally, for example, but not limited to wheat, soybean, corn, and the like. Similarly, the foodstuffs may include foods derived from animals or products of animals, for example, but not limited to meats, milk, eggs and the like, for example but not limited to from cows, pigs, lambs, chicken, fish, seafood and the like. Any plant or any animal that may be consumed as a food may be considered a foodstuff within the context of the present invention. In addition, the term "foodstuff' is meant to include any food component, or group or mixture of food components that is processed physically or chemically for example, but not limited to by cooking, curing, preserving, fermenting, pasteurizing, canning, sterilizing, irradiating and the like.

By the phrase "proteins in the foodstuff which are antigenic/immunogenic in a subject" it is meant one or more proteins in a foodstuff which may be bound by one or more antibodies in a subject having a pathological condition. The one or more antibodies in a subject that bind to a foodstuff may be present in the serum of a subject with the pathological condition.

By the term "pathological condition" it is meant an unnatural condition or disease which is detrimental to the subject if left untreated. In this manner the pathological condition may be clinical or subclinical. A subject with a subclinical pathological condition may be asymptomatic at a particular time, but may develop clinical signs of the pathological condition later on: In an embodiment of the present invention, the subject or group of subjects may comprise diabetes as a pathological condition. In an alternate embodiment of the present invention, the subject or group of subjects may comprise celiac disease as a pathological condition. In another embodiment of the present invention the subject or group of subjects may comprise both diabetes and celiac disease as pathological conditions.

By the term "subjects" it is meant any mammalian subject, for example, but not limited to rat, mouse, hamster, guinea pig, rabbit, chimpanzee or human. In a preferred embodiment, the subject is a human.

By the term "processing the foodstuff' it is meant separating proteins contained in the foodstuff by any method known in the art, for example, but not limited to 1D electrophoresis, 2D electrophoresis, chromatography, for example, but not limited to gel filtration, anion exchange, cation exchange, affinity chromatography, hydrophobic interaction, reverse phase and the like. In a preferred embodiment, the foodstuff is processed by 2D gel electrophoresis to separate proteins in the foodstuff, for example, but not limited to as described in the Examples. In this manner, a majority of the protein components in the foodstuff are separated from each other.

The processing of a foodstuff may also comprise one or more other processing steps including, but not limited to dialysis, extractions for example, alkali, acid, alcohol or organic chemical for example, but not limited to chloroform, methylene chloride, ethanol, methanol, butanol or a combination thereof, multiphase extractions, precipitations, or any combination thereof. In an embodiment of the present invention, which is not meant to be limiting in any manner, the method comprises a chromatographic step, extraction, precipitation or dialysis step prior to an electrophoresis processing step, for example, but not limited to two dimensional electrophoresis.

According to an alternate embodiment there is provided a method of screening foodstuffs to identify antigenic/immunogenic proteins common in at least two subjects, or groups of subjects wherein each subject or group of subjects comprise different pathological conditions, the method comprising the steps of
a) processing the foodstuff to produce separated proteins;
b) screening the separated proteins from step a) with a first antibody containing mixture derived from one or more subjects having a first pathological condition;
c) screening the separated proteins from step a) with a second antibody containing mixture derived from one or more subjects having a second pathological condition;
d) comparing proteins binding to the first antibody containing mixture with proteins binding to the second antibody mixture to identify proteins common in at least two subjects, or groups of subjects with different pathological conditions, the proteins also being present in the foodstuff.

Several variations of the method as disclosed above may also be employed in the present invention. For example, the step of processing the foodstuff in step a) may be performed in duplicate or higher, for example, but not limited to be under identical conditions. The steps of screening as described in b) and c) may each be performed on distinct samples of processed foodstuffs, preferably processed under identical conditions to ensure that all antigenic sequences in the foodstuff are available for binding by each antibody containing mixture. Further, one or more control subjects may be employed in the method of the present invention to further aid in the identification of proteins that are antigenic/immunogenic in a subject and that are present in the foodstuff.

The method of the present invention may further comprise a step of isolating and optionally sequencing the one or more proteins that are antigenic/immunogenic in the subject and present in the foodstuff. Further, the method of the present invention may comprise subjecting the one or more proteins to mass spectroscope, or epitope mapping, for example, but not limited to as described in the Examples section. It is also contemplated that the proteins or peptides may be characterized according to how they separate when subjected to one or more processes, for example, but not limited to eletrophoresis or the like.

### Modified Foods and Foodstuffs

There is disclosed foodstuffs, for example, but not limited to plants, animals, or processed foodstuffs therefrom, that are modified to reduce or eliminate proteins which are antigenic/immunogenic in a subject or group of subjects comprising a pathological condition. Alternatively, the foodstuffs may be modified to reduce or eliminate antigenic/immunogenic epitopes of foodstuff proteins which are anttgenic/immunogenic in a subject or group of subjects. In an embodiment of the present invention there is provided a foodstuff modified to reduce or eliminate Glb1, isoforms of gliadin proteins, or a diabetogenic epitope thereof. Proteins other than those listed above are also meant to be included by the present invention.

Foodstuffs may be modified to reduce or eliminate proteins by a variety of methods. For example, a plant or animal foodstuff may be genetically modified to "knockout" a gene of interest or portion thereof encoding a protein. Such methods are well known to those of skill in the art in molecular biology. Alternatively, techniques of RNAi may be employed to reduce or eliminate proteins. For example, but not wishing to be limiting in any manner, a region of the WP5212 cDNA clone may be amplified from cv. AC Barrie and inserted into the RNAi silencing vector, pHellsgate which has been used to transform wheat cells.

It is also contemplated that the foodstuffs comprising proteins may be processed to remove or reduce proteins that may be antigenic/immunogenic in a subject, for example, but not limited to by chemical, heat or protease treatment. Such treatments may modify or hydrolyze proteins at specific sequences and may destroy epitopes in proteins which are antigenic/immunogenic in a subject.

### Wheat protein diets can modulate diabetes outcome

Animals fed a non-purified, defined, mainly wheat-based NTP-2000 diet showed the highest incidence of diabetes (n=6 experiments, total of 169 rats, 65.3 ± 14.9 %, Figure 1). When comparing only defined, isocaloric and isonitrogenous semipurified diets with amino acids from wheat gluten or hydrolyzed casein, there were more cases of diabetes in BBdp rats fed wheat protein (WP) diets (n=12 experiments, total of 282 rats, 50.6 ± 11.1%) compared with BBdp rats fed a protective hydrolysed casein (HC) diet (n=14 experiments, total of 322 rats, 18.8 ± 10.6% Figure 1; ANOVA/LSD, p< 1 x 10⁻⁵). When fed to diabetes-prone BB rats, diets in which wheat gluten was the sole protein source induced nearly three times as many cases of diabetes as a hydrolyzed casein-based diet (Figure 2). To analyze as many potential diabetes-related wheat proteins as possible, more than one million recombinant phage from a wheat cDNA expression library were translated and screened with pooled sera from diabetic rats. Positive clones were subjected to nucleotide sequencing. One of the clones termed WP5212 was bound strongly by antibodies in sera from diabetic rats. Nucleotide and translated BLAST searches of Genbank ( NCBI. (2002) in the World Wide Web at ncbi.nlm.nih.govBLAST/, National Center for Biotechnology Information, National Library of Medicine, Bethesda, MD, USA) and TIGR Wheat Gene Index (TIGRWheatDatabase. (2001) in the World Wide Web at tigr.org/tdb/tagi/, Institute for Genomic Research, Rockville, MD, USA) databases revealed high similarity at the nucleotide and translated amino acid level with the wheat storage globulin protein, Glbl. Clone WP5212 contained a 1890 base pair (bp) open reading frame (ORF), including 95 bp of the *LacZ* gene. It shared 90% identity across 1387 nucleotides with the *Triticum aestivum* wheat storage protein (Glb1) gene (Acc. No. M81719.1). The expected translated amino acid sequence was 629 amino acids in length and shared 80% identity across 642 amino acids with the *T. aestivum* wheat storage protein (Acc. No. AAA34269.1), Glb1. IgG reactivity against Glb1 was strain-specific, highest in overt diabetic, lower in asymptomatic BB rats and lowest in non-diabetes-prone BBc rats.

Clones WP12111 and WP23112 also exhibited reactivity when the cDNA was translated and screened with pooled sera from diabetic rats. The open reading frame for cDNA clone WP12111 was 789 bp coding for a putative product of 262 amino acids. The nucleotide sequence shared 96% identity across 138 nucleotides with clone CNW03PL453 ITEC CNW from a wheat powdery mildew resistant line library (Acc. No. BE401554) and 63% identity across 144 amino acids with an unknown *Arabidopsis thaliana* protein (Acc. No. AAK25945).

Clone WP23112 had an ORF of 624 bp coding for a putative product of 207 amino acids. WP23112 shared 96% identity across 542 nucleotides with the BAC clone T16L24 from *A. thaliana* DNA chromosome 3 (Acc. No. 6899943) and 62% identity across 62 amino acids with the gene product, a putative *A. thaliana* protein (Acc. No. CAB75463.1). Clone WP23112 also shared-100% identity across 511 nucleotides with a clone from a Brevor mature wheat embryo ABA library (Acc. No. WHE0606).

### Diabetic rats have increased frequency and intensity of antibody reactivity to wheat proteins

Antibody reactivity to translated sequences from a wheat cDNA library, for example, but not limited to cDNA clones WP5212, WP12111 and WP23112 was assayed by screening with serum antibodies from individual diabetic (n=7), asymptomatic (n=10) and control (n=9) BB rats (Figure 2, panel A and B). Antibody reactivity was measured by densitometry and is reported as intensity/pixel. Antibody reactivity to WP5212 in diabetic rats was significantly higher than in asymptomatic (p=0.005) and control (p=10⁻⁶) rats. Asymptomatic BBdp rats also had increased antibody reactivity to WP5212 compared with control rats (p=0.0004). Diabetic rats also exhibited higher antibody reactivity to WP12111 than asymptomatic rats (p=0.02). Diabetic rats had increased antibody reactivity to WPCON compared with asymptomatic and control rats. Antibody reactivity in serum from BB control rats was not different among any of the proteins analysed, suggesting that this level represented nonspecific antibody reactivity.

The frequency of rats with antibodies to wheat proteins was determined (Figure 2, panel C). A positive antibody level was defined as an antibody reactivity value greater than the mean intensity plus 2 SD for WPCON screened with control serum. More diabetic (p=0.009) and asymptomatic (p=0.02) rats had antibodies to WP5212 than control rats.

Similar data from a human patient who has both type la diabetes and celiac disease indicated that antibodies from this highly wheat-sensitive individual bind strongly to the Glb1 protein, similar to the pattern we observed in diabetes-prone rats.

### Antibody reactivity to a Glb1 protein correlates with islet inflammation and damage

The autoimmune process involves progressive infiltration into the β-cell-containing core of the islets by mononuclear cells and macrophages, a process called insulitis. The severity and prevalence of insulitis or its sequelae (end stage islets) reflect the extent of damage in the pancreas. When sera from individual rats at different risk of developing diabetes were used, IgG reactivity against the Glb1 clone showed a remarkably close correlation with overall islet infiltration and damage (insulitis rating), as well as inflammation of individual islets. To determine if antibody reactivity to the cloned wheat proteins correlated with damage to the target tissue, the proportion of islets infiltrated with mononuclear cells was calculated, as well as the mean insulitis score. A relationship with diabetogenesis was considered to occur when both percent infiltration (degree of inflammation) and mean insulitis score showed a significant correlation with antibody intensity on the dot blots. Diabetic rats had significantly fewer islets than both asymptomatic and control rats. Diabetic rats had a higher percent of infiltrated islets and mean insulitis score compared with both asymptomatic (p=0.02 and p=0.0001) and control (p= 10⁻⁶ and p< 10⁻⁷) rats. In asymptomatic rats, the percent of infiltrated islets was higher, as was the mean insulitis score compared with control rats (p=0.0001 and p=0.002). A positive correlation was observed between antibody intensity to WP5212 and percent of infiltrated islets (Figure 3, panel A, r = 0.81, p = 10⁻⁶) and mean insulitis score (Figure 3, panel B, r = 0.78, p = 3 x 10⁻⁶). These results demonstrated not only a strong immune reaction against the Glb1 protein in wheat-fed, diabetes-prone BB rats, but also a close link with the diabetogenic process in the target tissue.

In patients with type 1 diabetes, the presence of autoantibodies to either GAD or islet antigen (IA)-2 has been shown to be closely correlated with *in situ* pancreatic islet inflammation (insulitis) and/or hyperexpression of MHC class I antigens in islets ( Imagawa, A., et al., (2001) Diabetes 50, 1269-1273.). Similarly, antibodies from BBdp and diabetic rats showed strong reactivity to the Glb1 protein, and this immunoreactivity correlated closely with the destructive immune process that targets the pancreatic islet β-cells in the pancreas. The close correlation between antibody reactivity to Glb1 and islet inflammation in BB diabetes-prone and diabetic rats, represents a new association between a previously unidentified wheat antigen and the target tissue. The fact that higher immunoreactivity to Glb1 was observed in patients compared with HLA-DQ matched non-diabetic children suggests that wheat may also be involved in the pathogenesis of human type 1 diabetes.

### Increased humoral immune reactivity to low molecular weight wheat proteins in pre-diabetic rats

To examine whether differences in antibody binding to wheat proteins were associated with the development of disease, Western blots of wheat gluten proteins were probed with serum obtained prospectively at 50 and 70 d from BB rats at different risk of developing diabetes. Western blots of wheat proteins showed antibody reactivity increased with age in BBdp rats (Figure 4, panel A). At day 50 the level of antibodies in asymptomatic and pre-diabetic rats was similar. Compared with animals that remained asymptomatic, higher signal intensity was detected for wheat proteins around 46 kDa (p=0.02, Figure 4 panel B) in prediabetic animals at approximately day 70. At necropsy, animals with overt diabetes had stronger reactivity to 36 kDa wheat proteins compared with asymptomatic rats (p=0.006). Blots probed with BB control rat serum at 1:600 showed low antibody binding to wheat proteins (data not shown). The frequency of rats reacting to these wheat proteins did not differ when comparing BBc, BBdp or overt diabetic animals.

### 1D and 2D Western blots show increased IgG binding to wheat proteins; Glb1 protein is bound by antibodies from patients but not controls

1D Western blots were used to investigate antibody binding to wheat proteins (Figure 5, panel A). Signal intensity for several proteins, for example, but not limited to the 33 kDa proteins was higher in patients than in controls in 19 of the 23 case-control comparisons (about 83%).

2D Western blots of wheat proteins probed with pooled sera from the same patients also showed IgG antibody binding to several wheat proteins (Figure 5, panel C). As in the case of diabetic BB rats, binding of antibodies to wheat proteins was widespread and more intense compared with controls. Wheat storage globulin, Glb1, consists of two subunits with a molecular weight of 49 kDa (pI 6.6) and 35 kDa (pI 6.9) (Marcone, M. F., et al., (1998) Food Chemistry 62, 27-47). One of the proteins bound by antibodies from diabetic children (but not controls) displayed a MW of 50 kDa and pI of 6.5. When the nature of this protein was determined using LC-MS/MS, it was found to have peptides homologous to both Glbl and WP5212. The expected (theoretical) peptide fragments of Glb1 and WP5212 and the experimental fragmentation detected by mass spectrometry are shown in Figure 6.

The prospective Western analysis showed a marked humoral response to certain low molecular weight (36 and 46 kDa) wheat proteins, particularly in animals that later developed overt diabetes. These bands are similar in size to the 35 and 49 kDa subunits of Glb1. Higher antibody binding to the 33 kDa band was present in 83% of diabetic children. This indicates a broad response to wheat proteins for example, but not limited to Glb1.

2D blots also showed higher antibody binding in diabetic children to several other wheat proteins (Figure 5, panel A and C). Glb1 was among these proteins, but absent in the 2D blots probed with control serum in keeping with the result of the 1D analysis (Figure 5, panel A). Without wishing to be bound by theory these results support the interpretation that diabetic patients have unique patterns of immune reactivity, some of which include for example, but not limited to Glb1. Increased peripheral blood T cell reactivity to wheat proteins was seen in 24% of newly diagnosed patients with type 1 diabetes, compared with only 5% of non-diabetic controls (Klemetti, P., et al., (1998) Scand J Immunol 47,48-53). Without wishing to be bound by theory, these data are consistent with the proposition that wheat antigens are a target of inappropriate immune responses in certain individuals who are genetically susceptible to develop autoimmune diabetes.

In order to identify additional diabetogenic wheat proteins, additional proteomic analyses of IgG antibody reactivity to wheat proteins subjected to 2D electrophoresis and Western blotting (See attached Figures 7 and 8) were performed. Blots were probed with serum from 26 T1D patients (age 25.4 ± 8.1 years) and 22 controls (24.9 ± 4.5 years). Only 1 out of 26 patients and 1 out of 22 controls was tissue transglutaminase positive suggesting the majority of subjects did not have subclinical celiac disease. T1D patients had stronger and more frequent reactivity to several WG proteins, for example, but not limited to 36-42 kDa proteins. LC-MS/MS analysis tentatively identified some of these proteins as gliadin isoforms. Three of these proteins were more frequently antigenic in T1D patients (Figure 8, filled spots, p≤0.05). Two of these proteins were identified as the γ-gliadin isoforms and a third protein is as yet unidentified, but is in a region of the blot known to contain other gliadin isoforms. On average, about 12% of controls and 41% of T1D patients reacted with the identified gliadin isoforms. With respect to the unidentified candidate wheat protein, none of the control subjects and 19% of T1 D patients showed antibody reactivity to this spot. Thus, analysis of spot frequency revealed that immune reactivity to specific wheat gluten proteins is increased significantly in a relatively large subset of T1D patients.

An analysis of wheat reactivity in two T1D children (8 y) showed reactivity to several wheat proteins, for example, but not limited to gliadin isoforms. One of these proteins was α/β-gliadin A-II precursor. This suggests that increased immune reactivity to certain gliadin isoforms and gliadin proteins is present in young patients and in those with diabetes of long duration.

Proliferation of T cells in response to wheat gluten was investigated using a recently described method of cell labeling with CFSE (Turcanu et al., J Clin Invest 111: 121:908-916, 2003). This method was developed to monitor proliferation of cells that represent a small proportion of the total population in peripheral blood. When cells that are labeled with CFSE divide, they lose half of the fluorescent label, and this process is repeated with each cell division. Therefore, cells that are proliferating undergo more divisions and become CFSE^{low}. This method enabled us to monitor chymotrypsin-treated WG stimulation of PBMC.

Without wishing to be bound by theory, these results suggest that a subset of T1D patients exists that is WG-responsive. This is higher than the frequency reported by Klemetti et al., who found 24% WG-responders using standard proliferation assays Scand.J.Immunol. 47:48-53, 1998. They observed gluten-induced proliferation more frequently in newly diagnosed patients whereas most of our patients were insulin-treated and with diabetes of longer duration. Thus, without wishing to be bound by theory or limiting in any manner, the sensitivity of the CFSE method may be an important factor in detecting WG-responsive subjects.

The preliminary data suggest that wheat reactivity is enhanced in a large subset of T1D patients compared with controls. If wheat causes or promotes diabetes in some individuals, it should be possible to either modify it to be less diabetogenic, avoid it in the diet or develop tolerizing regimes so the risk for wheat-induced T1D is minimized

Although it is possible to identify individuals at high risk for T1D, there is no safe intervention or treatment to offer them at this time. Dietary protein modification could be a safe, economical and effective means of preventing the development of islet autoimmunity and diabetes.

### EPITOPE MAPPING OF DIABETES-RELATED WHEAT PROTEINS

The antigenic epitopes of diabetes related wheat proteins, for example, but not limited to WP5212 (Glb-1) may be performed as described in Example 11. Without wishing to be limiting in any manner or bound by theory, defining the antigenic epitopes of WP5212 may be useful for a number of reasons: 1) epitope peptides may be easier to express; 2) epitope peptides can be synthesized; 3) epitope peptides can be used to immunize animals for the production of WP5212 specific antibodies; 4) epitope sequences may be homologous to self proteins providing evidence for cross-reactivity and potential molecular mimicry; and 5) antigenic epitopes may be more indicative of disease outcome and/or state.

To determine the antigenic epitopes of WP5212, a library of random, overlapping inserts expressed by transformed cells was screened by colony immunoscreening by using serum from an anti-Glb1 antibody positive patient with both type 1 diabetes and celiac disease. Bacterial colonies expressing immunoreactive peptides were selected and plasmid inserts were sequenced by using primers complementary to flanking regions of the cloning site in pSCREEN T-vector. The amino acid sequences from expressed WP5212 peptides were deduced. One clone contained a WP5212 fragment spanning nucleotides 937-967 of the original WP5212 clone was expressed in the correct reading frame with the T7 gene 10 fusion protein. It translates to a 10 amino acid sequence (EEQLRELRRQ (SEQ ID NO:1 )), which shares 100% (10/10) identity with the expected translated protein sequence of WP5212 (amino acids 309-318). It shares 90% (9/10) identity and 100% (10/10) positives with the protein sequence of wheat storage globulin Glb1; there is a conserved change from Q to E at position 10.

Of particular note, the Glbl epitope shares 90% (9/10) identity and 100% (10/10) positives with the protein sequence for desmin from human, cow, chicken and pig and there is a conserved amino acid change from Q to E at position three. It also shares 80% (8/10) identity and 100% (10/10) positives with the protein sequence for desmin from mouse, rat and hamster; there is a conserved amino acid change from Q to E at position three and L to M at position four.

Desmin is a marker of activated pancreatic stellate cells, which are involved in the development of fibrosis in chronic, alcohol induced and autoimmune pancreatitis (Bachem, M.G., et al., Gastroenterology, 1998. 115(2): p. 421-32; Haber, P.S., et al., Am J Pathol, 1999. 155(4): p.1087-95; Apte, M.V., et al., Gut, 1999. 44(4): p. 534-41). Pancreatic stellate cells have also been associated with fibrosis in acinar tissue during diabetes development (Taniyama, H., et al., J Vet Med Sci, 1999. 61(7): p. 803-10; Fehsel, K., et al., Lab Invest, 2003. 83(4): p. 549-59; Bach, J.F., Endocr Rev, 1994. 15(4): p. 516-42.) During an inflammatory process such as insulitis, immune mediators including interferon-γ, tumor necrosis factor α or production of nitric oxide can damage or destroy p-cells as well as neighboring cells (Bach, J.F., Endocr Rev, 1994.15(4): p. 516-42.). This 'bystander death' can result in the release of normally sequestered antigens from cells (Bach, J.F., Endocr Rev, 1994. 15(4): p. 516-42.). Without wishing to be bound by theory, it may be that stellate cells present in the islets or in surrounding acinar area are being destroyed in this manner resulting in antibody production to stellate cell proteins such as desmin and thus the homology between the Glbl peptide and desmin may therefore represent a form of molecular mimicry.

The antigenic epitope of WP5212 also shares 80% (8/10) identity and 100% (10/10) positives with the protein sequence for vimentin, an intermediate filament protein found in cells of mesenchymal origin. There is a conserved amino acid change from Q to E at position three and L to M at position four. The homology is between WP5212 and vimentin from mouse, rat, hamster, viper and puffer fish.

To determine the three-dimensional structure WP5212, the translated amino acid sequence was submitted to SWISS-MODEL (see Example 11). The SWISS-MODEL program superimposes the sequence of interest onto related solved three-dimensional structures from the RCSB Protein Databank (PDB). The amino acid sequence for WP5212 was aligned with template three-dimensional structures of canavalin from Jack bean *(Canavalia ensiformis;* PDB identification 2CAV and 2CAU) and beta-conglycinin from soybean *(Glycine max;* PDB identification 1IPJ and 1IPK). The expected three-dimensional structure of WP5212 based on these templates can be seen in Figure 9. Without wishing to be limiting or bound by theory, the antigenic epitope sequence appears to form part of an alpha-helix on an external hydrophilic portion of the protein as shown in (Figure 10).

### EXPRESSION OF DIABETOGENIC EPITOPES AND PROTEINS COMPRISING DIABETOGENIC EPITOPES

In an embodiment of the present invention there is provided a method of producing one or more diabetogenic epitopes or proteins comprising one or more diabetogenic epitopes in a transgenic cell comprising the steps of transforming the transgenic cell with a nucleotide construct comprising a promoter functional in the cell, the promoter driving the expression of a nucleotide sequence encoding a diabetogenic epitope or protein comprising a diabetogenic epitope. In an aspect of an embodiment, the transgenic cell may be a bacterial cell, for example, but not limited to an *E.coli* cell, an insect cell, a yeast cell, a plant cell in culture, a transgenic plant, a mammalian cell, for example, but not limited to a rat, mouse, goat or human cell.

The present invention will be further illustrated in the following examples, which are not meant to limit the scope of the invention in any way.

### EXAMPLE 1: Human Blood Samples

Blood samples for serum were obtained from Finnish children newly diagnosed with type 1 diabetes but not yet treated with insulin (n= 23; mean age 9.8 + 3.4 yr.) and non-diabetic control children (n=37; mean age 9.9 + 3.5 yr.), matched for age, sex and HLA-DQ MHC class II haplotype. Permission for blood sampling and ethics approval were obtained from the local ethics committee at the University of Turku.

### EXAMPLE 2: Animals

Male and female diabetes-prone BioBreeding (BBdp) and control BB rats (BBc) were obtained from the Animal Resources Division of Health Canada. The animals are maintained in laminar flow protected cages under specific pathogen-free conditions. The mean incidence of diabetes in BBdp rats from this colony fed a standard cereal-based diet (Rao, G. N. (1996) Fundam Appl Toxicol 32, 102-108) has remained constant over the past 5 years at 65.3 ± 14.9 % (mean ± Standard deviation (SD)). This colony is directly descended from the original diabetic rats discovered at BioBreeding laboratories near Ottawa in 1974 and transferred to Health Canada in 1977. The colony is not completely inbred, but has remained a closed colony for the past 25 years and recent genotyping for selected markers indicates the animals are about 80% identical at the DNA level. These animals carry the same mutation at the *Iddml*/*lyp* locus as BB/W rats that is attributable to a frameshift deletion in a novel member of the Immune-Associated Nucleotide (IAN)-related gene family, Ian5 (MacMurray, A. J., et al. (2002) Genome Res 12, 1029-1039). BBc rats are derived from an early subline of animals from the original BB rat colony that does not spontaneously develop diabetes. Tests in sentinel animals indicate the colony is antibody-free with respect to Sendai virus, pneumonia virus of mice, rat corona virus/sialodacryoadenitis virus, Kilham rat virus, Toolan's H-1 virus, reovirus type 3, and *Mycoplasma pulmonis.* Animals were weaned at 23 days of age, caged in banks of 30 wire-bottom cages, and given free access to food and water. The principles of laboratory animal care as described by the Canadian Council on Animal Care were followed.

Animals were tested twice weekly for glucose in urine using Testape (Lily, Toronto, Ontario) after 60 days of age. Those with a value greater than 2+ were fasted overnight, and blood glucose in tail blood was measured the next morning using a glucometer. Diabetes was diagnosed when fasting blood glucose was greater than about 11.1 mmol/l. Diabetic animals were killed within 24 h of diagnosis by exsanguination while under anesthesia with 3% halothane in oxygen.

### EXAMPLE 3: Insulitis Scores

All histological analyses were performed on coded samples. Hematoxylin and eosin stained sections of pancreas fixed in Bouin's solution were evaluated at 100× magnification, and confirmed at 200x magnification using an Axiolab microscope (Zeiss, Mississauga, Ontario). Subjective overall rating of pancreatic islet inflammation insulitis (Hoorfar, J., Scott, F. W., and Cloutier, H. E. (1991) J Nutr 121, 908-916) was performed using the following scale: 0, normal islet appearance; 1, infiltration in islet periphery only; 2, infiltration concentrated in islet periphery with infiltration in the islet core; 3, infiltration concentrated in one third of the islet core; 4, infiltration concentrated in up to one half of the islet core; 5, end stage islets with widespread β-cell destruction and/or core filled with infiltrating mononuclear cells. The mean of 10 islets per animal was used for an overall insulitis score. Inflammation of the islets was also measured as the percent of infiltrated islets.

### EXAMPLE 4: DIETS

### NTP-2000 (NTP) diet

The NTP-2000 diet (Zeigler Bros., Gardners, PA), is an open formula (the percentage composition is known), nonpurified diet for rodents developed by the U.S. National Toxicology Program of the National Institute of Environmental Health Sciences. NTP-2000 does not contain any milk protein. This is a mainly plant-based (milk-free) diet with wheat as the major component (37%), followed by corn, soybean meal, alfalfa meal, oat hulls, fish meal and cellulose. The diet contains approximately 14.6% protein, 8.2% fat, 9.9% crude fiber, 52% carbohydrate, 10.7% moisture; the remainder is native and added micronutrients. The NTP-2000 diet used in these studies was irradiated, and contained low levels of chemical and microbial contaminants (Rao, G. N. (1996) Fundam Appl Toxicol 32, 102-108).

### Wheat Protein (WP) diet

WP semipurified diets were made up of 22.5% wheat gluten (ICN Biochemicals, Cleveland, OH), 50.2% corn starch, 12.0% sucrose, 5.0% corn oil, 5.0% fiber (Solka-Floc), 3.5% AIN-76 (or AIN-93G) mineral mix (ICN), 1.0% AIN-76A (or AIN-93G) vitamin mix (ICN), supplemented with 0.2% choline bitartrate, 0.02% DL-methionine, 0.5% L-lysine, and 0.08% L-threonine to compensate for low sulfur amino acids in wheat proteins.

### Hydrolyzed casein (HC) diet

HC diets contained 51.0% corn starch, 12.0% sucrose, 20.0% casein hydrolyzate (Pancase S enzymatic hydrolysate, Redstar Bioproducts, Mississauga, Ontario), 7.0% soybean oil, 5.0% fiber, 3.5% AIN-76 (or AIN-93G) mineral mix, 1.0% AIN-76A (or AIN-93G) vitamin mix, 0.2% choline bitartrate, and 0.3% L-cystine. Both semipurified diets (WG and HC) were isocaloric and isonitrogenous.

### EXAMPLE 5: Wheat cDNA library construction and probing for antigenic proteins

Total RNA was isolated (Verwoerd, T. C., et al. (1989) Nucleic Acids Res 17, 2362) from hard red spring wheat, AC Barrie, provided by Dr. V. Burrows, Eastern Cereal Oilseed Research Centre, of Agriculture and Agri-Food Canada, Ottawa. Caryopses were harvested at approximately 10-20 d after pollination, total RNA was prepared and sent to Stratagene (La Jolla CA) to construct a ZAP Express^{®} Custom cDNA library. The cDNA was inserted into the *Eco*R I/*Xho* I cloning site in the amino-terminal region of the *lacZ* gene in the ZAP Express vector (Stratagene).

XL1-Blue-MRF' *Escherichia coli* were infected with 3.5 x 10⁴ pfu per plate (150 mm x 15 mm) of phage from the wheat ZAP Express Custom cDNA library following the manufacturer's instructions (Stratagene). Protein expression was induced by the addition of 15 µl of 2 M isopropyl-1-thio-β-D-galactopyranoside per 600 µl of *E. coli.* Plaque lifts were performed and the nitrocellulose membranes were screened following the manufacturer's instructions (Stratagene, La Jolla CA). The primary antibody (diluted 1:200 in SMP-TBS) consisted of pooled sera from 7 diabetic BB rats fed a wheat protein (WP) diet from weaning. The BB rat antibodies were pre-absorbed with *E*. *coli* phage lysate. The secondary antibody, alkaline phosphatase-conjugated AffiniPure goat anti-rat IgG, Fcγ fragment specific antibody (Jackson Immuno Research Laboratories Inc., West Grove PA), was diluted 1:5000 in SMP-TBS. Antibody binding was detected using alkaline phosphatase development solution (100 mM Tris-HCl, pH 9.5, 100 mM NaCl, 5 mM MgCl₂) containing nitroblue tetrazolium chloride (0.3 mg/ml) and 5-bromo-4-chloro-3-indolyl phosphate (0.15 mg/ml). Positive clones were detected as dark purple plaques and cored from the agar.

The agar plugs were placed in 500 µl of SM buffer (100 mM NaCl, 8 mM MgSO₄·7H₂O, 50 mM Tris-HCl (pH 7.5), 0.01% (w/v) gelatin) containing 20 µl chloroform and stored at 4°C. Screening was repeated until the positive phage reached clonality. Single clone excision was performed to allow *in vivo* excision and recircularization of the cloned insert, according to the manufacturer's instructions (Stratagene). Resistance to kanamycin indicated the presence of the pBK-CMV phagemid.

Phagemid DNA was prepared for sequencing using a Plasmid Midi Kit (Qiagen, Mississauga ON). The cDNA inserts were sequenced at the University of Ottawa Biotechnology Research Institute on a 373 Stretch sequencer (Applied Biosystems, Foster City CA) using standard T3 forward and T7 reverse primers. For clone WP5212, internal primers were designed to sequence the full cDNA insert (Forward: 5'-ACCACGGGTTCGTCAAGG-3' (SEQ ID NO:10), Reverse: 5'-AACACCTCCTGCACCTCC-3' (SEQ ID NO:11). Nucleotide and translated BLAST (Altschul, S. F., et al. (1997) Nucleic Acids Research 25, 3389-3402) searches of the Genbank (NCBI. (2002) in the World Wide Web at ncbi.nlm.nih.govBLAST/, National Center for Biotechnology Information, National Library of Medicine, Bethesda, MD, USA) and TIGR Wheat Gene Index (TIGRWheatDatabase. (2001) in the World Wide Web at tigr.org/tdb/tagi/, Institute for Genomic Research, Rockville, MD, USA) databases were performed for each sequence.

### EXAMPLE 6: Probing wheat clones for antibody reactivity using serum from individual rats fed WP-based diets

Serum (diluted 1:200 in SMP-TBS) from individual diabetic (n=7), asymptomatic (no clinical symptoms of diabetes by 150 d; n=10) BBdp, and BBc (n=9) rats was used to screen the wheat clones in the same manner as for the library screening. Densitometric analysis of regions of interest on nitrocellulose blots of wheat clones was performed using a Kodak Digital Science™ image station 440CF. The mean intensity/pixel for each region of interest was tabulated. A clone was randomly chosen from the library to represent background antibody binding. This clone, WPCON, had an ORF 366 bp long and an expected expression product size of 121 amino acids. WPCON shared 91 % identity across 326 nucleotides with barley ascorbate peroxidase mRNA *(Hordeum vulgare,* Acc. No. AF411228.1) and shared 96% identity across 86 amino acids with the ascorbate peroxidase protein (*H. vulgare,* Acc. No. AAL08496.1).

### EXAMPLE 7: 1D Western immunoblotting of wheat proteins

Proteins were extracted from wheat gluten powder (ICN) using lysis buffer as described previously (Gorg, A., Postel, W., and Gunther, S. (1988) Electrophoresis 9, 531-546). Samples were electrophoresed in 10% SDS-PAGE gels (Laemmli, U. K. (1970) Nature 227, 680-685), transferred to nitrocellulose, and blocked with 5% (w/v) skim milk powder in Tris-buffered saline (SMP-TBS, pH 7.5). Blots were incubated with sera diluted in SMP-TBS: 1:600. Samples were from rats at different risk of diabetes and fed WP diet: control BBc (n=10), asymptomatic BBdp (no clinical symptoms of diabetes by 120 d, n=7) and pre-diabetic BBdp animals (developed overt diabetes before 120 d, n=7 or animals with overt diabetes, BBd). Sera from individual patient (n=23) and non-diabetic HLA-DQ-matched control children (n=37) was diluted 1:50. Following 5 x 5 min washes with TBS containing 1% (v/v) Tween 20, the membrane was exposed to horseradish peroxidase-conjugated goat anti-rat IgG (Fcγ-fragment specific; Cedarlane Laboratories, Hornby, Ontario) or rabbit anti-human total IgG antibody (Dako), diluted 1:2000 with SMP-TBS. Bands were visualized using enhanced chemiluminescence (ECL) according to the manufacturer's instructions (ECL-Western blotting detection, Amersham Pharmacia Biotech, Buckinghamshire UK) and quantified by densitometry. Digital images of the Western blot films were acquired using the Kodak Digital Science™ image station 440CF (Rochester NY) and analyzed using the Kodak Digital Science™ 1D image software (New Haven CT).

### EXAMPLE 8: 2D Western immunoblotting of Wheat Proteins

150 µg of wheat gluten proteins in lysis buffer were added to the IEF buffer (4% CHAPS, 7 M urea, 2 M thiourea, 40 mM Trizma base, 2 mM tributylphosphine and 0.4% Bio-lyte 3/10 (BioRad)) and applied to rehydrated Ready Strips (BioRad) with an immobilized linear pH gradient (IPG) from pH 3-10. Wheat proteins were focused at 21 °C for a total of 100,000 Volt hours on the Protean IEF cell (BioRad), reduced with 20 mg/ml of dithiothreitol and alkylated with 25 mg/ml iodoacetamide. Proteins were separated in the second dimension in 10% SDS-PAGE gels by electrophoresis at 30 mA for 15 min and 60 mA for 2 h, transferred to nitrocellulose membrane and blocked using SMP-TBS overnight at 4°C. Serum was pooled from the 23 patients and 37 controls, diluted 1:500 in SMP-TBS buffer, and used to probe 2D Western blots (1 hour at 4°C). The secondary antibody, rabbit anti-human total IgG conjugated with horseradish peroxidase (Dako), was diluted to 1:2000 with SMP-TBS buffer and incubated with the membranes for 30 min at 4°C. Antibody binding was visualized using ECL as recommended by the manufacturer (Amersham Pharmacia, Baie d'Urfe, Québec) and analyzed using 2D analysis software (PDQuest, BioRad, Toronto, Ontario).

### EXAMPLE 9: Mass spectrometry analysis

2D gels of wheat gluten proteins were stained with a non-fixing silver stain (Gharahdaghi, F., et al.. (1999) Electrophoresis 20, 601-605.). Excised gel plugs were digested overnight at 37°C with 200 ng of modified sequencing grade trypsin (Promega) in the ProGest™ automatic digester (Genomic Solutions, Ann Arbor MI) as described (Gharahdaghi, F., et al., (1999) Electrophoresis 20, 601-605.). Rapid capillary LC-MS/MS (capLC-MS/MS) was performed using a Waters CapLC liquid chromatograph (Waters, Milford MA) coupled to a Q-TOF 2 mass spectrometer (Micromass, Manchester UK) with an electrospray ionization interface. The digest extracts were redissolved in 5% (v/v) acetonitrile, 0.5% acetic acid and 10 µL was loaded onto a 0.3 x 5 mm C₁₈ micro pre-column cartridge (Dionex/LC-Packings) for each analysis. Rapid peptide elution was achieved using a linear gradient of 5 to 60% acetonitrile, 0.2% formic acid in 6 minutes (flow rate of 1 µL/min). The mass spectrometer was operated in data dependent acquisition mode.

### EXAMPLE 10: Statistical analysis

Comparisons between sample populations were made using one-way ANOVA and Scheffé or LSD post hoc tests (STATISTICA Version 4.5, StatSoft Inc., 1993, Tulsa OK). Fisher's Exact test (2-tailed) was used to compare the frequency of individuals with antibody reactivity to wheat proteins. Pearson Product-Moment correlation was used to determine r and p values. Survival analysis using the log-Rank test was used to compare the effect of different diets on diabetes incidence (STATISTICA).

### EXAMPLE 11: Construction of the WP5212 epitope library.

The Novatope Library Construction System (Novagen, Madison, WI) for characterizing B cell epitopes within antigenic polypeptides was used to map sites of antibody binding within WP5212. Plasmid DNA was introduced into *E*. *coli* NovaBlue (DE3) cells, and transformants were selected with ampicillin. DNase I digestion of the WP5212/pET17b expression construct containing the complete ORF of WP5212 was performed using the DNase Shotgun Cleavage kit (Novagen) following the manufacturer's instructions. The DNase I digestion reaction was set up by adding 10 µg of WP5212/pET17b plasmid DNA to DNase I (0.0015, 0.001, 0.0007 or 0.0005 U/µl), DNase I buffer (0.05 M Tris-HClpH7.5, 0.05 mg/ml BSA), 10 mM MnCl₂ in a total volume of 10 µl. The reactions were incubated at room temperature for 10 min, after which 2 µl of 6X Stop Buffer (100 mM EDTA, 30% glycerol and tracking dye) was added. Samples were analyzed by agarose gel electrophoresis on 2% gel. The samples containing DNA fragments ranging in size from 50-150 bp were pooled and run on a 2% agarose gel. The band corresponding to 50-150 bp fragments was excised from the gel and equilibrated by adding 1X volume of the gel of 10X agarose buffer (100 mM Bis Tris-HCl pH 6.5, 10 mM Na₂EDTA) to make 1% gel. The gel was melted at 65°C for 10 min. The sample was cooled to 42°C and incubated with molten agarose with 2 U per 200 µl 1% agarose for 1 h. The DNA solution was extracted sequentially with one volume TE-buffered phenol, one volume of 1:24:1 phenol:chloroform:isoamyl alcohol (phenol:CIAA) and 1 volume CIAA. The DNA was precipitated by adding 0.1 volume of 3 M NaOAc and 2 volumes ethanol. The sample was placed at -70°C for 1 h. It was centrifuged at 12,000 X g for 15 min, washed once with 1 volume of 70% ethanol, centrifuged at 12,000 X g for 5 min and the pellet was dried. The DNA was resuspended in 30 µl TE buffer. The DNA concentration was determined at A₂₆₀.

The resulting oligonucleotides (ranging 50-150 bp in length) were blunt-ended and tailed with a single dATP using the Single dA Tailing Kit (Novagen). The DNA ends were blunted by combining 1 µg of the DNA with flushing buffer (0.05 M Tris-HCl pH 8.0, 5 mM MgCl₂, 0.1 mg/ml BSA), 0.1 mM dCTP, dGTP and dTTP, 1 mM dATP, 5 mM DTT and 1 U T4 DNA polymerase in a total volume of 25 µl. The reaction was mixed gently and incubated at 11°C for 20 min. The reaction was stopped by heating to 75°C for 10 min. A single 3' dA residue was added to the blunt ends by adding 10X dA tailing buffer (100 mM Tris-HCl pH 9.0, 0.5 M KCl, 0.1% gelatin, 1% Triton X-100) and 1.25 U of *Tth* DNA polymerase to the entire flushing reaction and bringing the reaction volume to 85 µl. A positive dA tailing control was set up by adding 100 ng of a positive control 36mer to flushing buffer (0.05 M Tris-HCl pH 8.0, 5 mM MgCl₂, 0.1 mg/ml BSA), 0.1 mM dCTP, dGTP and dTTP, 1 mM dATP, 10X dA tailing buffer and 0.75 U Tth polymerase in a total reaction volume of 42.55 µl. The reactions were incubated at 70°C for 15 min. One volume of CIAA was added, the sample was vortexed for 60 s and centrifuged at 12,000 X g for 1 min. The aqueous phase was added to a fresh tube.

The oligonucleotides were ligated into the pSCREEN T-vector. The ligation reaction was made by adding 6 ng sample DNA to 5 mM DTT, 0.5 mM ATp, 50 ng pSCREEN T-vector, 2 U T4 DNA ligase in a total reaction volume of 10 µl. The sample was mixed gently and incubated overnight at 16°C. The DNA was transformed into NovaBlue (DE3) competent cells following the protocol provided. In brief, 1 µl of the ligation reaction was added to 20 µl of cells and stirred gently. The samples were placed on ice for 30 min. The samples were heated for 30 s at 42°C and then placed on ice for 2 min. Cells were grown for 1 h at 37°C with shaking at 250 rpm. 50 µl of each transformation was spread on LB agar plates containing 50 µg/ml ampicillin and 15 µg/ml tetracycline. The plates were inverted and incubated overnight at 37°C.

### Screening the WP5212 epitope library

The library of random, overlapping inserts expressed by transformed cells was screened by colony immunoscreening with anti-WP5212 antibody positive serum from a highly wheat sensitive female patient with both type 1 diabetes and celiac disease (age 26 years). The epitope library was plated at a density of approximately 1000 colony forming units (cfu) on LB plates containing 50 µg/µl ampicillin and grown overnight at 37°C. The plates were overlaid with nitrocellulose filters (VWR). The orientation of the filter on the plate was marked by an 18 gauge needle dipped in India ink. After one min of contact, the membranes were carefully lifted off and placed in a chloroform vapor chamber for cell lysis. The chamber was sealed with Saran wrap and left for 15 min at room temperature. The membranes were removed from the chamber and placed colony side up on a piece of Whatman paper saturated with colony denaturing solution (20 mM Tris-HCl pH 7.9, 6 M urea, 0.5 M NaCl) for 15 min at room temperature. The membranes were placed in TBST containing 5% w/v nonfat skim milk powder and incubated with shaking for 30 min. The membranes were washed twice for 15 min each with TBST and placed in the primary antibody diluted in blocking buffer for 30 min. Colonies were immunoscreened for protein expression using anti-WP5212 positive serum from a T1D/CD patient (1:2000). The membranes were washed 3 times for 10 min each with TBST and placed in secondary antibody, anti-human IgG conjugated to alkaline phosphatase (1:20,000) for 30 min. The membranes were washed three times for 10 min each with TBST and placed in AP developer. Dark purple colonies were determined to be positive.

### Identification of epitopes.

Bacterial colonies expressing immunoreactive peptides were selected and plasmid inserts were sequenced by using primers complimentary to flanking regions of the cloning site in pSCREEN T-vector. Positive colonies were picked and grown overnight at 37°C with shaking. Plasmid DNA was prepared using the Wizard Plus SV miniprep kit following the manufacturers instructions (Promega). Sequencing of the inserts was performed by the Ottawa Genome Centre DNA Sequencing Facility (Ottawa, ON). Insert sequences were aligned with the ORF WP5212 sequence using Align Plus 5, version 5.01 software from the Clone Manager Professional Suite (Scientific and Educational Software, Durham, NC).

### Protein modeling

The translated amino acid sequence of WP5212 was submitted to SWISS-MODEL (the World Wide Web at expasy.org/swissmod/SWISS-MODEL.html) for three-dimensional rendering (Peitsch, M.C., Bio/Technology, 1995. 13: p. 658-660; Peitsch, M.C., Biochem Soc Trans, 1996. 24(1): p. 274-9; Guex, N. and M.C. Peitsch, Electrophoresis, 1997. 18(15): p. 2714-23).

### EXAMPLE 12: Method of Screening cDNA library with Sera from T1D/CD patients:

To identify and characterize T1D-related wheat proteins, a large number, for example, but not wishing to be limiting, about 1 million recombinant wheat proteins from a wheat cDNA expression library are screened with IgG antibodies in pooled sera from patients with T1D and T1D/CD. Candidate clones are also screened with sera from two control groups (CD patients and healthy controls).

An approach similar to that used to identify and characterize Glb-1 and other proteins is employed to screen a wheat cDNA expression library with IgG antibodies in pooled sera from patients with T1D and T1D/CD. Sera from patients with CD alone is used to screen the clones identified by Group I and 2 sera to identify wheat antigens that are common to T1D, T1D/CD and CD patients. A control group of age and sex-matched subjects is included. To eliminate proteins that are not diabetes-related, candidate clones are probed with sera from the control group and positive clones are eliminated from further analysis. Clones identified by screening the library with pooled serum from T1D and T1D/CD are probed with antibodies from individual subjects in the four groups to determine what proportion reacts to each protein. Candidate clones are expressed in insect cells as described below.

### Wheat protein expression in insect cells

Glb1 (WP5212) is provided as an example. However, a person of skill in the art will understand that other diabetogenic proteins and peptides may be expressed in such cells.

*IPLB-Sf21 insect cell line:* Insect *Spodoptera frugiperda* (Sf21, BD Biosciences) cells are maintained in Grace's insect cell culture medium, plus 10% fetal bovine serum (Sigma), penicillin (50 units/ml), and streptomycin (50 µg/ml) at 27°C. Insect *Trichoplusia ni* (High-Five™, Invitrogen, Carlsbad, CA) cells are maintained in Express-Five™ SFM medium at 27°C. For protein expression, High-Five cells are maintained in suspension cultures.

### PCR amplification and subcloning of the FLAG-tagged WP5212 into pBacPak9 transfer vector

The WP5212 sequence is amplified by PCR. The forward PCR primer is designed to include an *Eco* RI restriction enzyme site, a start codon followed by the FLAG™ (Sigma, Oakville, ON) epitope sequence in frame with the WP5212 cDNA. The reverse primer is designed to include an *Eco* RI restriction enzyme site (Sigma Genosys). The 1.9 kb WP5212 PCR product is cloned into the pCR^{®}2.1 plasmid using the TOPO^{®} TA-cloning kit (Invitrogen). Plasmid DNA from clones containing inserts is prepared using the Wizard Plus SV Miniprep kit (Promega). WP5212 is cloned into the *Bam* HI/*Xho* I restriction enzyme sites of the pBacPak9 transfer vector (BD Biosciences). The presence of WP5212 is confirmed by restriction digests with *Eco* RI and sequencing using Bacl and Bac2 primers (BD Biosciences).

### Generation of a recombinant BacPak6 baculovirus Glbl (WP5212) expression system

Transfected insect cells with the recombinant virus containing Glbl (untagged) has been performed and the protein has been identified in lysates of High Five cells by Western blot analysis with BB rat serum. Purification of Glbl may be performed according to a variety of methods known in the art. Recombinant baculovirus is generated using the Bac-PAK baculovirus expression system (Clontech). Sf21 cells are co-transfected with WP5212-pBakPAK9 and BacPAK6 viral DNA (Clontech) using Bacfectin (Clontech) following the manufacturer's instructions. Recombinant virus is isolated, propagated and amplified in Sf21 cells. High-Five cells are cultured in Express-Five™ SFM medium (Invitrogen) at 27°C and infected with the recombinant virus. After infection and protein production, cells are harvested and lysed and insoluble fractions are removed by centrifugation. The expression of FLAG-tagged WP5212 is confirmed by Western blot analysis of insect cell lysate. Samples are diluted in 2X sample loading buffer and electrophoresed in 10% SDS-PAGE gels and electrotransferred to nitrocellulose membranes. For immunoblotting, membranes are incubated in blocking buffer, followed by incubation in mouse anti-FLAG™ M2 monoclonal antibody (Sigma) diluted in blocking buffer. Membranes are washed, incubated in the secondary antibody, rabbit anti-mouse polyvalent immunoglobulins monoclonal antibody conjugated to alkaline phosphatase (Sigma) diluted in blocking buffer. Antibody binding is detected using alkaline phosphatase solution. Recombinant protein is purified by anti-FLAG™ M2 agarose (Sigma) affinity chromatography. The FLAG tag is removed from purified recombinant protein with enterokinase and enterokinase is removed using the enterokinase removal kit (Sigma).

Disease-specific clones are isolated from the library by screening with sera from T1D and T I D/CD patients. Screening the clones with control serum reveals non-specific clones and those that are positive with CD serum aids differentiation of CD-specific versus T1D-specific proteins (or identify shared antigenic proteins). These clones are sequenced and identified on the basis of homology to known wheat proteins. Recombinant wheat proteins for use in *in vitro* T-cell assays may be produced to further elucidate the role of these proteins in T1D:

### Identify TID-related proteins by proteomic analysis of wheat gluten protein extracts

An analysis of antibody reactivity to wheat using 2D Western blots may be performed in parallel with the library screening. WG protein extracts are probed with IgG antibodies from each individual patient and control as described above. Candidate proteins may be characterized using a capillary liquid chromatograph coupled to a QTOF-2 mass spectrometer (LC-MS/MS), or other chromatography and/or mass spectroscopy system.

### 2-D electrophoresis and Western blot (Isoelectric Focusing (IEF))

Immobilized pH gradient strips (IPG; BioRad; broad range pI 3-10) are rehydrated overnight with 150 µg of WG proteins in BioRad ReadyPrep Sequential Extraction Reagent 3 and 0.002 M Tributylphosphine. Proteins are then focused for 95,000 Vh on a Protean IEF Cell apparatus (Bio-Rad) at 23°C.

### SDS-PAGE

The focused wheat proteins on the IPG strip are reduced with dithiothreitol and alkylated with iodoacetamide. The proteins are resolved on 10% polyacrylamide gradient gels at 40 mA in a Tris-glycine-SDS running buffer.

### Western immunoblotting and Mass Spectroscopy

Wheat proteins are transferred onto nitrocellulose membranes. Non-specific antibody binding is blocked with 5% skim milk powder in Tris-buffered saline at 4°C. Membranes are probed with serum from individual patients or controls (1:500 dilution) for 1 hour at 23°C. An HRP-anti-total IgG secondary antibody (1:50,000 dilution), is used to detect antibody-bound wheat proteins using Enhanced Chemiluminescence (Amersham). Results are analyzed using 2D analysis software (PDQuest, BioRad). Proteins are resolved on a polyacrylamide gel and visualized using a non-fixing silver stain. Gel plugs with proteins of interest are excised from the gel for trypsin digestion and mass spectrometry (MS) analysis, as described previously. Rapid capillary LC-MS/MS is performed using a CapLC Liquid chromatograph (Waters, Milford, MA) coupled to a Q-TOF2 MS. Database searching is performed automatically using Mascot™ (Matrix Science, London, U.K.), which searches for homologous sequences in the peptide MS/MS spectra files and protein and nucleotide sequence databases. In cases where the spectra cannot be matched, sequencing is performed manually; sequences are used to search the SwissProt, NCBI and other protein sequence databases using MS-Seq (Protein Prospector™, Mass Spectrometry Facility, UCSF, San Francisco, CA) or NCBI's BLAST searching algorithms.

### Produce recombinant candidate wheat proteins and determine antigenic/immunogenicity in human T cells

Candidate wheat proteins may be expressed in insect or other cells and purified. Candidate proteins and peptides are tested in human PBMC and wheat-reactive T cell lines from a variety of T1D patients and HLA matched controls for ability to stimulate T cell proliferation, alter gene and protein expression of inflammatory mediators or response to T1D autoantigens such as insulin or GAD.

This research (1) identifes specific wheat proteins that are differentially immunoreactive in T1D (and T1D/CD) patients, (2) determines what proportion of T1D patients show increased antibodies and T cell responses to these proteins, and (3) identifies the T cells being stimulated.

A subset of a control patient group and T1D patient group are HLA matched and analyzed for T cell reactivity to WG, and other food antigens, controls and identified candidate proteins or gliadin peptides. Analysis of small populations of antigen-specific T-cells using flow cytometry of cells labeled with CFSE is performed. Cells that proliferate are characterized by a decreasing fluorescent signal; these cells can be isolated by FACS on the basis of this characteristic, allowing further analysis to confirm specificity and determine phenotype. Our studies demonstrate that this method can be used for the isolation of WG-responsive T-cell populations.

### CFSE labeling and cell culture.

Blood is collected in heparin-treated vacutainers, diluted 1:1 in sterile PBS and PBMC are isolated by density-gradient using Histopaque-1077 (Sigma Diagnostics). As described (Turcanu et al., J. Clin invest 111:1065-1072, 2003) PBMC are resuspended in 1 ml sterile PBS and stained for 10 min at 37°C with 2µM of CFSE. Cells are washed once with RPMI-1640 supplemented with 10% (v/v) pooled human serum, 2.0 µM L-glutamine, 50 mM 2-mercaptoethanol, 100 U/ml penicillin and 100 µg/ml streptomycin (RP-10) followed by a second wash in X-Vivo 15 medium. PBMC are plated at 2x10⁶ /2 ml X-Vivo-15 in 24 well-plates and stimulated with medium (control), 2.5 µg/ml PHA, 1 µM ovalbumin protein (OVA), 2µg/ml β-lactoglobulin or varying concentrations of chymotrypsin-digested WG (12.5, 6.25 or 3.1 µg/ml).

### Proliferation of CFSE labeled T cells to WG and generation of WG-reactive T cell lines.

Both CD4⁺ and CD8⁺ T cells play a role in T1D and thus both of these populations may be examined. 1x10⁵ CFSE-labeled cells cultured in the presence of chymotrypsin-WG, gliadin peptides, other diabetogenic peptide (or protein) or control antigens (ovalbumin, lactalbumin, PHA (positive control), KLH, (negative control) are stained with human anti-CD3-ECD to monitor the proliferation of all T lymphocytes (CD4 and CD8 lymphocytes). Double-stained T cells are analyzed for cell division by FACS. CD3⁺T cells show reduced CFSE signal due to proliferation (CD3⁺CFSE^{low}) and are gated as WG-reactive T cell population. To generate WG-reactive T cells lines, WG-expanded cells are harvested and sorted by flow into CD3⁺CFSE^{low} and CD3⁺CFSE^{high} cells. Single cell clones are generated from CD3⁺CFSE^{low} population by flow sorting using 96 well plates with one cell per well. Single clones are cultured in medium containing 10 IU/ml of hIL-2. After expansion, single cell clones are tested against candidate proteins or immunoreactive gliadin (or other) peptides to examine clone WG-specificity.

### Phenotyping of PBMC.

To compare PBMC from T1D patients to cells from controls, 1x10⁶ PBMC are resuspended in 100 µl FACS buffer and stained with anti-CD 19, anti-CD83 and anti-CD3 (Pharmingen) to monitor the expression of B cell, DC and T cell surface markers respectively on resting cells.

### Specificity and phenotype of WG-reactive T cells.

2 x 10⁴ cells / well of WG-reactive T cells obtained by flow sorting plus 2 x 10⁵ irradiated autologous APCs are plated in 96-well plates and stimulated with varying concentrations of chymotrypsin treated-WG (or test antigens) in medium alone or with control antigens added. After 5 d, the cells are pulsed with 1 µCi per well of ³H-thymidine (Amersham Pharmacia Biotech) for an additional day. Cells are harvested onto glass fiber filters (Packard) and radioactivity is measured. To determine the proportion of CD4 and CD8 cells comprising WG-reactive T cells (CD3⁺CFSE^{low}), sorted T cells are double stained with anti-CD4-PE and anti-CD8-Cy5 and analyzed by flow cytometry.

### HLA typing.

1x10⁶ PBMC are cultured in medium in the presence of 2.5 µg/ml PHA. At d 5, 5x10⁶ cells are harvested and sent to the Biochemistry Section, Ottawa Hospital for HLA DR and DQ typing.

### WG-induced gene expression in TlD PBMC.

Gene expression in WG-reactive T cells obtained from T1D patients is analyzed by an array technique. 5x10⁶ sorted WG-reactive T cells are stimulated with PMA/ionomycin for 6 hours. Cells are harvested for total RNA preparation using Trizol (Life Technologies). 2-5 µg total RNA are analyzed for inflammatory cytokine, cytokine receptors, chemokines and growth factor gene expression using the Q series of common human cytokines and interleukin receptor microarray membranes (SuperArray Bioscience Corporation, Frederick, MD). Superarray membranes contain up to 96 cDNA fragments from genes associated with specific biological pathways.

### Cytokine profile of WG-reactive T cells.

2x10⁴ cells/well of WG-reactive T cells from T1D patients obtained as above, plus 2 x 10⁵ irradiated autologous APCs in 200 µl are stimulated with varying concentrations of WG or other test antigen in X-Vivo 15 medium or control antigens. At d7 post-stimulation, cell free supernatants are harvested for Th1/Th2 cytokine profile analysis by capture ELISA. ELISA experiments are performed using OptEIA human IFN-γ (Th1-associated cytokine) and IL-5 (Th2-specific cytokine) kits (BD Biosciences) using a modified protocol.

Flow cytometry facilitates phenotype determination of the WG-reactive immune cells (CD4, CD8). Without wishing to be limiting or bound by theory, the predominant T-cell phenotype may be CD4⁺ IFN- γ secreting cells as in CD. The cytokine expression data will facilitate the determination of whether the Th1/Th2 cytokine balance of the wheat-responsive cells favours an inflammatory response (Th1 predominant), (Th2) or is tolerizing (Th3). As part of the specificity testing, the capacity of T1D autoantigens, GAD and insulin, to stimulate proliferation and cytokine production may be evaluated. This study will facilitate identification of specific, potentially diabetogenic proteins/peptides responsible for triggering the abnormal immune response in T1D patients and aid in the determination as to which type(s) of immune cells (CD4, CD8) in T1D patients are WG-reactive.

The present invention contemplates a method of screening a subject for T-cell reactivity toward proteins or peptides that comprise one or more diabetogenic epitopes comprising isolating T-cells from the subject; contacting the T-cells with one or more proteins or peptides comprising one or more diabetogenic epitopes and monitoring the proliferations of the T-cells. One or more controls may also be employed in the method of screening.

### EXAMPLE 13: Expression of WP5212 in Sf21 insect cells and increased antibody reactivity to WP5212 in patients with type 1 diabetes.

### Patient study group.

Patients and healthy control subjects were recruited in accordance with the guidelines of the Ottawa Hospital Research Ethics Board and the Children's Hospital of Eastern Ontario Ethics Board. Permission for blood sampling was given by study subjects with informed consent. Serum was tested for IgA antibodies to the celiac disease-associated autoantigen tissue transglutaminase (tTG). Peripheral blood mononuclear cells (PBMC) were isolated from blood using Histopaque-1077 (Sigma Diagnostics). HLA typing for DRB 1 and DQB 1 was performed on genomic DNA by the Ottawa Hospital General Campus Biochemistry Lab.

### IPLB-Sf21 insect cell line.

Insect Spodoptera frugiperda (Sf21) cells were grown at 27 °C in complete Grace's insect cell culture medium (supplemented; Gibco, Burlington, ON) containing 10% fetal bovine serum (Sigma), penicillin (50 units/ml; Sigma) and streptomycin (50 µg/ml; Sigma).

### Recombinant WP5212 expression.

12.0 x 10⁶ log phase insect cells were plated in a 150 cm² flask and incubated at 27°C for 2 hours. The cells were infected with WP5212-baculovirus or parental BacPAK6 virus at a multiplicity of infection (MOI) = 10. Uninfected cells acted as a negative control. The cells were incubated for 2 days at which point they were harvested and centrifuged for 5 min at 1,000 x g. The pellet was washed twice with PBS and resuspended in 100 µl PBS. The cells were lysed by sonication. Samples were quantified using the BioRad DC Protein Assay, following the manufacturer's protocol.

### Peptide synthesis.

Antigenic WP5212 peptides were predicted by Sigma-Genosys (The Woodlands, TX) technical services. Two WP5212 specific peptides were synthesized for the purpose of polyclonal WP5212 antisera production (Sigma-Genosys). Peptide 1 was a 77% pure 16 residue peptide (CRDTFNLLEQRPKIAN (SEQ ID NO:12)) and peptide 2 was a 51 % pure 15 residue peptide (RGDEAVEAFLRMATA (SEQ ID NO: 13)). Both were conjugated to the carrier protein Keyhole-limpet hemocyanin for immunization. The purity was determined by mass spectral and HPLC analyses performed by Sigma-Genosys.

### Polyclonal antisera production.

Antibody production was performed by Sigma-Genosys. Pre-immune serum was collected from two rabbits, after which they were each co-immunized with 200 µg of peptides 1 and 2 in Complete Freund's Adjuvant (day 0). The rabbits were co-immunized with 100 µg of peptides 1 and 2 in Incomplete Freund's Adjuvant on days 14, 28, 42, 56 and 70. Production bleeds from both rabbits were collected on day 40, 63 and 77. WP5212-specific polyclonal antibody production was assessed by 1D SDS-PAGE and Western blotting of WP5212-baculovirus infected insect cell lysate. Production bleeds were compared to pre-immune serum samples.

### 1D SDS-PAGE and Western blotting.

Protein samples were diluted in 2X SDS sample loading buffer and denatured by heating for 5 min at 100°C. Ten µg of total protein was added to each well. A prestained molecular mass marker was used (BenchMark Prestained Protein Ladder, Invitrogen, Burlington, ON). Protein samples were resolved by electrophoresis on 10% SDS-PAGE gels at 200 V in electrophoresis running buffer (25 mM Tris-base, 192 mM glycine, 0.01% SDS, 0.1 mM phenylmethylsulfonyl fluoride (PMSF, Sigma)). Proteins were electrotransferred from the gel to pure nitrocellulose membranes (pore size 0.45 µm, Bio-Rad) at a constant current of 300 mA (approximately 25 V) for 1 hour in transfer buffer (25 mM Tris-base, 192 mM glycine, 20% methanol, pH 8.3). The membrane was stained with Ponceau S red (Sigma) to ensure protein transfer to the nitrocellulose membrane. The membrane was washed in TBST for 4 x 5 min and placed in blocking buffer overnight at 4°C. The membranes were placed in primary antibody, serum diluted 1:200 in blocking buffer containing 0.09% sodium azide, for 1 hour with shaking. The membranes were washed 4 x 5 min each in TBST and placed in secondary antibody, goat anti-human IgG (Fc-specific) antibody conjugated to alkaline phosphatase (Sigma) diluted 1:20,000 in blocking buffer, for 1 hour. The membranes were washed 2 x 5 min with TBST and 2 x 5 min with TBS. Antibody binding was detected using alkaline phosphatase development solution containing nitroblue tetrazolium (0.3 mg/ml) and 5-bromo-4-chloro-3-indolyl phosphate (0.15 mg/ml). Densitometric analysis of bands of interest was performed using the Kodak Digital ScienceTM image station 440CF (Kodak Canada Inc.). For each blot, background optical density was measured and subtracted from the value for the band of interest. The band mean intensity/pixel was tabulated.

### Statistics.

Means were calculated and differences among sample populations were compared using the Student's t test or one-way ANOVA and LSD post hoc test. Fisher's exact test (two-tailed) was used to compare frequencies. All statistics were performed using STATISTICA Version 4.5 (StatSoft Inc., 1993, Tulsa OK).

### Expression of WP5212 in Sf21 insect cells.

To explore the relationship between the wheat storage globulin WP5212 and diabetes in humans, patients with diabetes and age and sex-matched healthy control subjects were screened for WP5212 antibodies. To semi-quantitatively measure antibody titer, a system was developed to standardize the quantity of protein screened. The identity of the over-expressed protein in recombinant WP5212-baculovirus infected Sf21 insect cells was confirmed by probing Western blots with WP5212 polyclonal antiserum produced in rabbits. Two rabbits were co-immunized with two WP5212-specific peptides. Pre-immune rabbit serum samples do not have WP5212 antibodies (Figure 11). The immunized rabbits developed WP5212 specific antibodies (Figure 11). WP5212 is expressed as doublet of 61 and 67 kDa in these insect cells.

### Patients with type 1 diabetes have increased antibody reactivity to WP5212.

The study group consisted of patients with type 1 diabetes (n = 26; mean age = 25 ± 8 years; mean age at T1D diagnosis = 13 ± 10; Table 3), celiac disease (n = 4; mean age = 28 ± 4 years), both diseases (n = 4; mean age = 30 ± 14 years; mean age at T1D diagnosis = 17 ± 5 years) or healthy control subjects (n = 21; mean age = 25 ± 6 years). The subjects participating in the study provided blood samples after giving informed consent. There were no significant differences in age at sampling, sex ratio or age at T1D diagnosis (where applicable) among the groups. The HLA haplotypes for all diabetic, celiac and T1D/CD patients, as well as 19 of 21 control subjects were determined (Table 4). Twenty-five of 26 type 1 diabetes patients and all control subjects were negative for antibodies to the celiac autoantigen tissue transglutaminase (tTG; Table 4). All patients with both T1D and CD were weakly positive or positive for tTG antibodies and two CD patients were weakly positive (Table 4).

1D SDS-PAGE and Western blotting of uninfected, BacPAK6 parental baculovirus or WP5212-baculovirus infected insect cell lysate was performed to determine whether patients developed disease-associated WP5212 antibodies (Figure 12, panels A to D). No non-specific binding was observed from the secondary antibody (data not shown). Antibody reactivity was determined by densitometric analysis. Subjects were designated WP5212 antibody positive when antibody reactivity was greater than the mean intensity plus 2 SD for WP5212 screened with healthy control serum (mean intensity/pixel + 2SD = 2363.6, indicated by the solid line on Figure 12, panel E and F). It was observed that, indeed, a subgroup of diabetic patients developed WP5212 antibodies with higher reactivity than observed in control subjects (p < 0.001, Figure 12, panel E and F).

Remarkably, the frequency of WP5212 antibody positivity was about 10 fold higher in the diabetic group than in the control group (4.8 vs. 42.3%, p = 0.006; Figure 12, panel E and F; Table 5). In addition, it was striking that the four patients with both T1D and CD displayed the highest mean antibody reactivity to WP5212 in comparison with healthy control subjects, as well as patients with T1D or CD (Figure 12 panel F). Also, because celiac disease is the result of an immune reaction to wheat proteins and could contribute to the production of WP5212 antibodies, WP5212 antibodies were evaluated in patients with CD alone. Two of four CD patients were WP5212 antibody positive and there was no difference in mean antibody reactivity between the WP5212 antibody positive diabetic and CD patients (Figure 12, panel F).

The incidence of CD is as much as 25 fold higher in T1D patients compared with the general population. Of patients with both T1D and CD, 90% develop T1D first, suggesting that after CD diagnosis individuals placed on a gluten-free diet are less likely to develop diabetes. Also, a large proportion of patients with autoimmune diabetes, as much as one third, have been shown to develop antibodies to the celiac disease associated autoantigen tissue transglutaminase (Bao et al., 1999. J Autoimmun 13: 143-8; Rewers et al., 2004. Endocrinol Metab Clin North Am 33: 197-214). The presence of anti-tTG antibodies could indicate subclinical CD and thus confound the finding of high WP5212 antibody levels specifically in T1D patients. Therefore, tTG antibodies were measured in all subjects studied (Table 4). No control subjects tested positive for autoantibodies to tTG. Two patients with CD tested weakly positive for tTG antibodies, likely reflecting recent dietary exposure to gluten proteins, and two patients were negative. All four of the T1D/CD patients were weakly positive or positive for tTG antibodies despite following a gluten free diet. The one weakly tTG antibody positive T1D/CD patient was a patient following a strict specific-carbohydrate, cereal-free diet. The persistence of tTG antibodies in these patients further suggests that they are at the extreme end of wheat sensitivity. Only one of 11 T1D patients with positive WP5212 antibody levels developed tTG autoantibodies (tTG antibody value 27; Table 4). The level of WP5212 antibody reactivity for this particular patient was the lowest among the group of WP5212 antibody positive T1D patients (data not shown).

Interestingly, the T1D patient that tested positive for tTG autoantibodies had the HLA-DR3/4, DQ2/* haplotype and thus shared genetic risk for both type 1 diabetes and celiac disease. The data suggest that antibodies to WP5212 could be used as a marker of a subset of T1D patients with wheat-related diabetes. Furthermore, the presence of strong WP5212 antibody reactivity in T1D patients could indicate susceptibility to the future development of CD.

Autoimmune diabetes and celiac disease share a number of HLA-associated risk genes. HLA-DQ2 and DQ8 are both associated with T1D and CD but at different frequencies: 90-95% of CD patients are HLA-DQ2 and 5-10% are HLA-DQ8 (Lango and Lindblom 1993. Eur J Immunogenet 20: 453-60; Farre et al., 1999. Dig Dis Sci 44: 2344-9) while up to 50% diabetic patients are HLA-DQ8/DQ2 heterozygotes (Melanitou et al., J Autoimmun 21: 93-8). The high coincidence of T1D and CD has been attributed to shared genetic risk. Therefore, it was of interest to determine whether the production of WP5212 antibodies in T1D patients was associated with a CD predisposing genetic background. Of the 11 WP5212 antibody positive T1D patients, four did not have a haplotype associated with CD (neither HLA-DQ2 nor DQ8 homo-or heterozygous; Tables 4 and 6). Six WP5212 antibody positive patients were HLA-DQ2 heterozygous and one was HLA-DQ8 heterozygous, both haplotypes associated with celiac disease. Also, the T1D patient with positive tTG antibody titre was HLA-DR3/4, DQ2/* (Tables 4 and 6). In short, 64% of the T1D patients that developed antibodies to WP5212 had haplotypes associated with celiac disease, while 36% did not.

The development of WP5212 antibodies may be a unique indicator of wheat-induced immune activation in a subset of susceptible individuals independent of CD genotype and tTG autoantibody status, indicating its potential use as a novel prognostic and/or diagnostic tool for wheat-related T1D. Also, WP5212 antibody development may represent a marker for later development of CD in T1D patients. WP5212 antibody reactivity could also be a marker of enhanced gut immune reactions to dietary wheat proteins or gut damage.

| **Table 3**. Study group characteristics. | | | | |
|---|---|---|---|---|
| **Subject type** | **N** | **Sex ratio (MIF)^{a}** | **Mean age ± SD (years)^{a}** | **Mean age ± SD at T1D diagnosis (years)^{a}** |
| T1D | 26 | 10/16 | 25 ± 8 | 13 ± 10^{b} |
| CD | 4 | 1/3 | 28 ± 4 | N/A |
| T1D/CD | 4 | 0/4 | 30 ± 14 | 17 ± 5^{b} |
| Control | 21 | 7/14 | 25 ± 6 | N/A |
| ^{a} No significant differences in sex ratio, age or age at T1 D diagnosis among the groups. | | | | |
| ^{b}Data for one patient missing | | | | |
| N/A: not applicable | | | | |

| **Table 4**. HLA haplotype and tissue transglutaminase autoantibody status of individual study subjects | | | | | | |
|---|---|---|---|---|---|---|
| **Group** | **ID** | **Sex (M/F)** | **Age (Y)** | **DR and DQ HLA Haplotype** | **tTG Ab^{a}** | **WP5212 Ab pos** |
| T1D | D1 | F | 23 | DRB1*03,*13, DR52, DQB1*02,*06 | 3 | Yes |
| | D2 | F | 20 | DRB1*01,*04, DR53, DQB1*03,*05 | <1 | |
| | D3 | F | 38 | DRB1*01;*03, DR52, DQB1*02,*05 | <1 | Yes |
| | D4 | F | 19 | DRB1*03,*04, DR52,DR53, DQB1*02,*03 | <1 | |
| | D5 | M | 22 | DRB1*04,DR53,DQB1*03 | <1 | Yes |
| | D6 | M | 21 | DRB1*04,DR53,DQB1*03 | <1 | |
| | D7 | M | 29 | DRB1*04,DR53,DQB1*03(DQ7),*03(DQ8) | <1 | Yes |
| | D8 | F | 8 | DRB1*0301,*0401,DR52,DR53,DQB1*0201 ,*0302 | <1 | |
| | D9 | F | 8 | DRB1*03,*04, DR53, DQB1*03 | <1 | Yes |
| | D10 | M | 22 | DRB 1*03(DR17),*04, DR52, DR53, DQB1*02 ,*03 | <1 | |
| | D11 | F | 22 | DRB1*03(DR17),DR52,DQB1*02 | <1 | |
| | D12 | M | 21 | DRB1*01,*04,DR53,DQB1*05,*03(DQ7) | <1 | |
| | D13 | F | 27 | DRB1*15,*04,DR53,DR51,DQB1*06,*03(D Q7) | <1 | |
| | D14 | F | 26 | DRB1*04,*13,DR52,DR53,DQB1*06,*03(D Q8) | <1 | |
| | D15 | M | 25 | DRB1*03,*12,DR52,DQB1*02,*03(DQ7) | <1 | Yes |
| | D16 | F | 18 | DRB1*03,*12,DR52,DQB1*02,*03(DQ7) | <1 | Yes |
| | D17 | M | 19 | DRB1*15,DR51,DQB1*06 | N/A | Yes |
| | D18 | F | 33 | DRB1*04*13,DR522,DR53,DQB1*06,*03(D Q7) | <1 | |
| | D19 | M | 30 | DRB1*03,*04,DR52,DR52,DQB1*02,*0305 | <1 | |
| | D20 | M | 23 | DRB1*03,*13,DR52,DQB1*06,*02 | <1 | |
| | D21 | M | 33 | DRB1*03,*04,DR52,DR53,DQB1*02,*0305 | 27 | Yes |
| | D22 | F | 25 | DRB1*15,*13,DR52,DR51,DQB1*06 | <1 | Yes |
| | D23 | F | 34 | DRB1*03,*04; DR52;DR53; DQB1*02,*03(DQ8) | <1 | |
| | D24 | F | 37 | DRB1*04; DR53; DQB1*03 | 2 | |
| | D25 | F | 29 | DRB1*03,*04,DR52,DR53,DQB1*02, *03(DQ8) | <1 | |
| | D26 | F | 41 | DRB1*04, *07; DR53; DQB1*03 (DQ8,9) | <1 | Yes |
| | | | | | | |
| CD | CD1 | F | 27 | DRB1*03,*07,DR52,DR53,DQB1*02 | <1 | |
| | CD2 | M | 33 | DRB1*01,*13,DR52,DQB1*05,*06 | 6 | |
| | CD3 | F | 25 | DRB1*15,*03,DR52,DR52,DR51,DQB1*06, *02 | <1 | Yes |
| | CD4 | F | 26 | DRB1*03(17),*13,DR52,DQB1*02,*03(DQ7 ) | 7 | Yes |
| | | | | | | |
| T1D/CD | D/CD1 | F | 28 | DRB1*0301,*0401,DR52,DR53,DQB1*0201 ,*0302 | 7 | Yes |
| | D/CD2 | F | 12 | DRB1*0101,*0301,DR52,DQB1*0501,*0201 | 16 | Yes |
| | D/CD3 | F | 43 | DRB1*03,*04,DR52,DR53,DQB1*02,*03 | >100 | Yes |
| | D/CD4 | F | 38 | DRB1*04,DR53,DQB1*03 | >100 | Yes |
| | | | | | | |
| Control | C1 | F | 28 | DRB1*03(DR17)*12,DR52,DQB1*02,*03 | <1 | |
| | C2 | F | 26 | DRB1*04,*08,DR53,DQB1*03,*04 | <1 | |
| | C3 | F | 38 | N/A | <1 | |
| | C4 | F | 22 | DRB1*13,*15,DR51,DR52,DQB1*06 | <1 | |
| | C5 | M | 23 | DRB1*03,*07,DR52,DR53,DQB1*02 | <1 | |
| | C6 | M | 24 | N/A | <1 | |
| | C7 | M | 29 | DRB1*15,*04,DR53,DR51,DQB1*06,*03(D Q7) | <1 | |
| | C8 | M | 28 | DRB1*07,DR53,DQB1*02,*03(DQ9) | <1 | |
| | C9 | F | 21 | DRB1*13,*15,DR51,DR52,DQB1*06 | <1 | Yes |
| | C10 | F | 22 | DRB1*03,*15,DR52,DR53,DQB1*02,*06 | <1 | |
| | C11 | F | 29 | DRB1*11,*12,DR52,DQB1*03 | <1 | |
| | C12 | F | 40 | DRB1*11,*14,DR52,DQB1*03(DQ7),*05 | <1 | |
| | C13 | M | 28 | DRB1*01,*07,DR53,DQB1*05,*02 | <1 | |
| | C14 | F | 23 | DRB1*15,*07,DR53,DR51,DQB1*06,*03(D Q9) | <1 | |
| | C15 | F | 24 | DRB1*03,*07,DR52,DR53,DQB1*02 | <1 | |
| | C16 | M | 22 | DRB1*07,*11,DR52,DR53,DQB1*02,*03 (DQ7) | <1 | |
| | C17 | F | 18 | DRB1*11,DR52,DQB1*03 (DQ7) | <1 | |
| | C18 | F | 23 | DRB1*16, *14,DR52,DR51,DQB1*05 | <1 | |
| | C19 | M | 26 | DRB1*01,*07,DR52,DQB1 *05,*02 | <1 | |
| | C20 | F | 18 | DRB1*01,*04,DR53,DQB1*05,*03 (DQ7) | <1 | |
| | C21 | F | 22 | DRB1*03, *11,DR52,DQB1*02, *03 | <1 | |
| ^{a} A tissue transglutaminase autoantibody value less than 4 is negative, a value between 4 and 10 is weakly positive, a value greater than 10 is positive. | | | | | | |
| N/A: not available | | | | | | |

| **Table 5.** Increased frequency of T1D and T1D/CD patients develop WP5212 antibodies in comparison with control subjects. | | | |
|---|---|---|---|
| **Subject type** | **Frequency** | **% Positive** | **p value vs. controls** |
| Control | 1/21 | 4.8 | - |
| T1D | 11/26 | 42.3 | 0.006 |
| CD | 2/4 | 50.0 | 0.06 |
| T1D/CD | 4/4 | 100.0 | 0.0004 |
| ^{a}Fisher's exact two-tailed test | | | |

| **Table 6**. Association of HLA haplotype and diabetes and/or celiac disease risk. Sensitivity of diabetes associated genotypes was determined using data from Lambert et al., 2004. J Clin Endocrinol Metab 89(8):4037-43 and that of celiac disease associated genotypes was determined using data from Margaritte-Jeannin et al. 2004. Tissue Antigens 63(6):562-7 | | | | | | | | | |
|---|---|---|---|---|---|---|---|---|---|
| **Diabetes Risk** | | | | | | | | | |
| Low | | Predisposing | | | | | | High | |
| DR15/* DQ6/* | DR34/* DQ3/* | DR4 DQ3 | DR3/4 DQ3/* | DR3/* DQ2/* | DR4/* DQ8/* | DR4 DQ8/* | DR3/4 DQ2/* | DR3 DQ2 | DR3/4 DQ2/8 |
| 2/11 | 0/11 | 1/11 | 1/11 | 5/11 | 0/11 | 1/11 | 1/11 | 0/11 | 0/11 |
| | | | | | | | | | |
| Low | | | Predisposing | | | | | | |
| **Celiac Disease Risk** | | | | | | | | | |

### EXAMPLE 14: Increased T Cell Immune Response To Wheat Proteins In Patients With Type 1 Diabetes

An experiment was performed to determine what proportion of T1D patients show abnormal.T cell responses to wheat proteins

### Subjects

T1D patients were recruited through physicians at the Ottawa Hospital and CHEO, Ottawa, Canada. The subjects have clinically proven T1D, CD/T1D or CD. Subjects are mostly young adults and a few children of both sexes between 8-43 years of age. The controls are healthy individuals in a similar age range (Table 7). Blood was obtained by venepuncture from patients and healthy controls with informed consent and the local ethics committee approved the study.

| **Table 7**. Description of subjects | | | |
|---|---|---|---|
| Group | n | Sex (f/m) | Mean age (range) |
| | | | |
| T1D | 28 | 19/9 | 25 (8-41) |
| CD, CD/T1D | 6 | 5/1 | 26 (25-43) |
| Control | 23 | 13/10 | 25 (18-41) |

### Isolation of mononuclear cells, tissue culture and antigen response

PBMC were isolated from blood by density gradient centrifugation over Ficoll (histopaque-1.077 Sigma). Cells were washed twice with Hank's buffer containing 20 mmol HEPES. PBMC (20 x 10⁶/ ml) were labeled with 2mM CFSE for 20 minutes and incubated at 37°C in 5% CO₂. Cells were washed twice with Hank's buffer containing 5% pooled human AB+ serum (Bioreclamation INC) and finally diluted in RPMI-1640 (Sigma R8758) containing 5% Human AB+ serum, 1 x L-glutamine (GIBCO 25030-081), 25 mM HEPES (GIBCO 15630-080), 50 mM β-mercaptoethanol and 1% antibiotic/antimycotic (GIBCO 15240-096). Cells were cultured with various concentrations of Wheat protein (chymotrypsin-treated, heat-inactivated wheat gluten, WP) (3.2-12.4 mg/ml), gliadin 10 mg/ml (Sigma), α-gliadin 33 mer 10 mg/ml, human GAD (hGAD) 10 mg/ml, insulin 10 mg/ml, ovalbumin (Ova) 1 mmol, Tetanus toxoid (TT) 2.7 LF/ml and PHA 5 mg/ml. After 2 days of culture, 10 IU/ml rhIL-2 was added to each well. On day 8, supernatants were harvested and cell proliferation was assessed using a CFSE-based, flow cytometric assay with results expressed as cell division index (CDI).

### HLA Typing

HLA DR and DQ haplotypes of subjects were characterized by PCR-based HLA class II tissue typing.

### Statistical analysis

Differences in CDI among patients and controls and CD patients were analyzed by the non-parametric Kruskal-Wallis test by using CDI = 8.77 as a cutoff limit for positive response to wheat protein. Significance of differences in frequencies was evaluated using Fisher's exact two-tailed test.

Results of the experiments performed are shown in Figures 13-16 and Table 8.

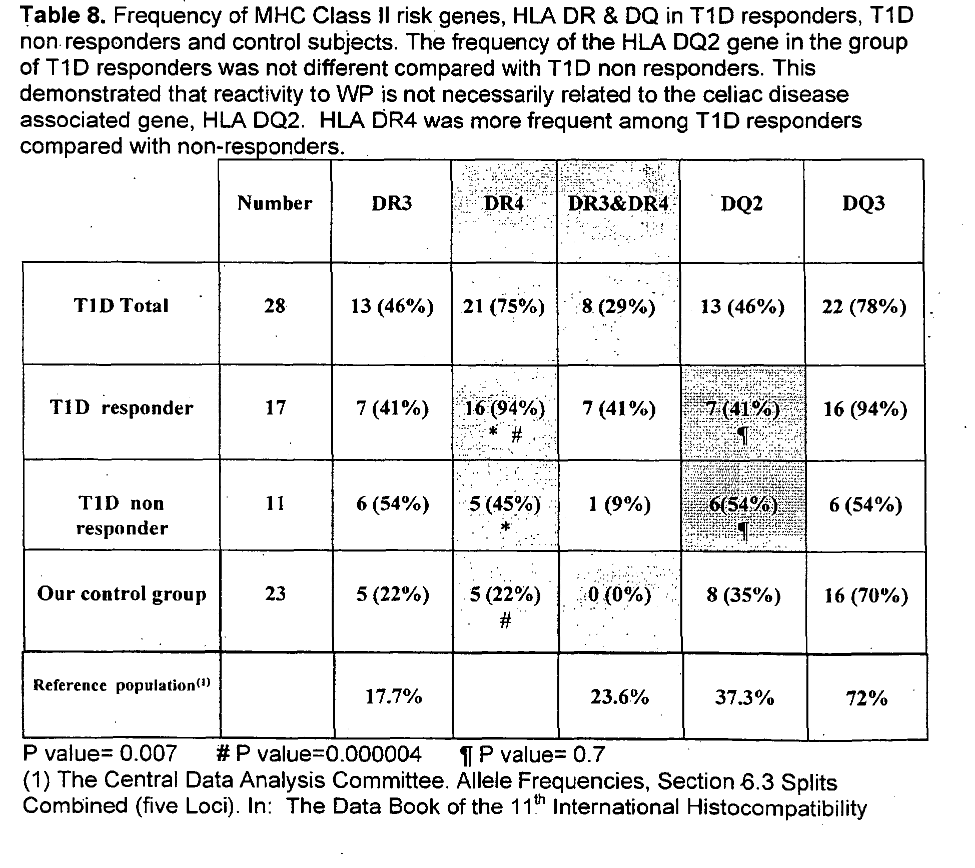

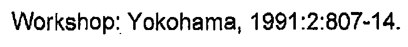

In T1D patients and control subjects, the proliferation of T cells in response to a panel of antigens was inventigated, reported as cell division index (CDI): wheat proteins, ovalbumin, T1D autoantigens (GAD and insulin) and tetanus toxoid using a CFSE proliferation assay. This method enabled an evaluation of the phenotype of responder cells and the degree of response.

The T1D patient group showed a higher mean CDI compared with control subjects whose responses were low and variable. In addition, significantly increased responses could be detected in some T1D patients to wheat gliadin, an α-gliadin 33-mer peptide, (a key celiac related antigen), and the T1D autoantigens, GAD and insulin. The wheat responders were defined as those with a CDI higher than the control group mean + 2 SD. The data indicates that half of T1D patients could be classified as wheat protein responsive.

Some MHC class II risk alleles are common for both T1D and celiac disease. Therefore, HLA DR and DQ haplotypes were analysed. Analyses in a subgroup of T1D patients indicated the frequency of the HLA DQ2 allele which is found in 95% of CD patients was not significantly different between the T1D responder patients and non responders. In contract, a positive response to WP was associated with HLA DR4. Thus, the results suggest there is an unusually high T cell reactivity to wheat in a substantial subset of T1D patients and this response is associated with the high risk diabetes gene, HLA DR4.

Collectively the results indicate that there is an unusually high T cell reactivity to wheat proteins in a large subset of T1D patients. Further, reactivity to WP in T1D patients is not necessarily related to the major celiac disease associated gene, HLA DQ2. Also, these data suggest that a positive response to WP in T1D patients is associated with HLA DR4.

The present invention has been described with regard to one or more embodiments. However, it will be apparent to persons skilled in the art that a number of variations and modifications can be made without departing from the scope of the invention as defined in the claims.

## Claims

1. An isolated amino acid sequence comprising a diabetogenic epitope, said diabetogenic epitope comprising EEQLRELRRQ (SEQ ID NO:1).

2. The isolated amino acid sequence of claim 1, wherein said amino acid sequence comprises SEQ ID NO:4.

3. The isolated amino acid sequence of claim 1 or 2, attached to a carrier protein, non-carrier protein, macromolecule or support.

4. The isolated amino acid sequence of claim 3, attached to a support, said support comprising at least one of a bead, a plate, a dish, a cover slip, a slide, a multiwell assay plate, and a bio-assay chip.

5. A nucleotide sequence encoding a diabetogenic epitope, wherein said diabetogenic epitope consists of the amino acid sequence defined by EEQLRELRRQ (SEQ ID NO:1) or comprises the amino acid sequence defined by SEQ ID NO:4.

6. The nucleotide sequence of claim 5, wherein said diabetogenic epitope comprises the protein defined by SEQ ID NO:4.

7. The nucleotide sequence of claim 5, comprising one or more regulatory sequences.

8. The nucleotide sequence of claim 5, comprising a cloning vector.

9. A nucleotide sequence complementary to the nucleotide sequence of claim 5.

10. An isolated antibody capable of binding to at least one of a diabetogenic epitope comprising EEQLRELRRQ (SEQ ID NO:1).

11. The isolated antibody of claim 10, said antibody comprising a monoclonal antibody.

12. The isolated antibody of claim 10, said antibody comprising an IgG antibody.

13. The isolated antibody of claim 10, said antibody produced in the serum of a non-human animal.

14. The isolated antibody of claim 13, wherein said animal is a diabetic animal.

15. A kit comprising at least one of:
a. a diabetogenic epitope comprising the sequence EEQLRELRRQ (SEQ ID NO:1),
b. a protein or peptide comprising a diabetogenic epitope, wherein said protein or peptide comprises the sequence EEQLRELRRQ (SEQ ID NO:1) or (SEQ ID NO:4),
c. a non-protein carrier or macromolecule comprising the diabetogenic epitope EEQLRELRRQ (SEQ ID NO:1) or SEQ ID NO:4,
d. a support comprising the diabetogenic epitope EEQLRELRRQ (SEQ ID NO:1) or SEQ ID NO:4,
e. a nucleotide sequence encoding a diabetogenic epitope that consists of the amino acid sequence defined by EEQLRELRRQ (SEQ ID NO:1) or comprises the amino acid sequence defined by SEQ ID NO:4, and
f. a nucleotide sequence complementary to a nucleotide sequence encoding the a diabetogenic epitope that consists of the amino acid sequence defined by EEQLRELRRQ (SEQ ID NO:1) or comprises the amino acid sequence defined by SEQ ID NO:4.

16. The kit of claim 15, further comprising at least one selected from the group consisting of a bead, a plate, a dish, a coverslip, a slide, a multi-well assay plate, a bioassay chip.

17. The kit of claim 15, further comprising at least one primary antibody capable of binding to the diabetogenic epitope EEQLRELRRQ (SEQ ID NO:1) or a protein comprising the diabetogenic epitope EEQLRELRRQ (SEQ ID NO:1).

18. The kit of claim 17, further comprising at least one secondary antibody capable of binding to the at least one primary antibody.

19. The kit of claim 17, further comprising at least one selected from the group consisting of a solution, a reagent, an enzyme.

## Patentansprüche

1. Isolierte Aminosäuresequenz, welche ein diabetogenes Epitop umfasst, wobei das diabetogene Epitop EEQLRELRRQ (SEQ-ID Nr. 1) umfasst.

2. Isolierte Aminosäuresequenz nach Anspruch 1, wobei die Aminosäuresequenz SEQ-ID Nr. 4 umfasst.

3. Isolierte Aminosäuresequenz nach Anspruch 1 oder 2, gebunden an ein Trägerprotein, ein Nichtträgerprotein, ein Makromolekül oder einen sonstigen Träger.

4. Isolierte Aminosäuresequenz nach Anspruch 3, gebunden an einen Träger, wobei der Träger mindestens eines aus einem Kügelchen, einer Platte, einer Schale, einem Deckglas, einem Objektträger, einer Assay-Platte mit mehreren Vertiefungen und einem Bioassay-Chip umfasst.

5. Nukleotidsequenz, welche ein diabetogenes Epitop codiert, wobei das diabetogene Epitop aus der durch EEQLRELRRQ (SEQ-ID Nr. 1) definierten Aminosäuresequenz besteht oder die durch SEQ-ID Nr. 4 definierte Aminosäuresequenz umfasst.

6. Nukleotidsequenz nach Anspruch 5, wobei das diabetogene Epitop das durch SEQ-ID Nr. 4 definierte Protein umfasst.

7. Nukleotidsequenz nach Anspruch 5, welche eine oder mehrere Regulatorsequenzen umfasst.

8. Nukleotidsequenz nach Anspruch 5, welche einen Klonierungsvektor umfasst.

9. Nukleotidsequenz, welche zu der Nukleotidsequenz nach Anspruch 5 komplementär ist.

10. Isolierter Antikörper, welcher an mindestens ein diabetogenes Epitop binden kann, welches EEQLRELRRQ (SEQ-ID Nr. 1) umfasst.

11. Isolierter Antikörper nach Anspruch 10, wobei der Antikörper einen monoklonalen Antikörper umfasst.

12. Isolierter Antikörper nach Anspruch 10, wobei der Antikörper einen IgG-Antikörper umfasst.

13. Isolierter Antikörper nach Anspruch 10, wobei der Antikörper in dem Serum eines Tieres erzeugt wird.

14. Isolierter Antikörper nach Anspruch 10, wobei es sich bei dem Tier um ein diabetisches Tier handelt.

15. Kit, welcher mindestens eines aus dem Folgenden umfasst:
a. ein diabetogenes Epitop, welches die Sequenz EEQLRELRRQ (SEQ-ID Nr. 1) umfasst,
b. ein Protein oder Peptid, welches ein diabetogenes Epitop umfasst, wobei das Protein oder Peptid die Sequenz EEQLRELRRQ (SEQ-ID Nr. 1) oder SEQ-ID Nr. 4 umfasst,
c. einen Nicht-Protein-Träger oder ein Makromolekül, welcher/-es das diabetogene Epitop EEQLRELRRQ (SEQ-ID Nr. 1) oder SEQ-ID Nr. 4 umfasst,
d. einen Träger, welcher das diabetogene Epitop EEQLRELRRQ (SEQ-ID Nr. 1) oder SEQ-ID Nr. 4 umfasst,
e. eine Nukleotidsequenz, welche ein diabetogenes Epitop codiert, das aus der durch EEQLRELRRQ (SEQ-ID Nr. 1) definierten Aminosäuresequenz besteht oder die durch SEQ-ID Nr. 4 definierte Aminosäuresequenz umfasst, und
f. eine Nukleotidsequenz, welche zu einer Nukleotidsequenz komplementär ist, die ein diabetogenes Epitop codiert, das aus der durch EEQLRELRRQ (SEQ-ID Nr. 1) definierten Aminosäuresequenz besteht oder die durch SEQ-ID Nr. 4 definierte Aminosäuresequenz umfasst.

16. Kit nach Anspruch 15, welcher ferner mindestens eines umfasst, das aus der Gruppe ausgewählt ist, die aus einem Kügelchen, einer Platte, einer Schale, einem Deckglas, einem Objektträger, einer Assay-Platte mit mehreren Vertiefungen und einem Bioassay-Chip besteht.

17. Kit nach Anspruch 15, welcher ferner mindestens einen primären Antikörper umfasst, der an das diabetogene Epitop EEQLRELRRQ (SEQ-ID Nr. 1) oder an ein Protein binden kann, welches das diabetogene Epitop EEQLRELRRQ (SEQ-ID Nr. 1) umfasst.

18. Kit nach Anspruch 17, welcher ferner mindestens einen sekundären Antikörper umfasst, der an den mindestens einen primären Antikörper bindet.

19. Kit nach Anspruch 17, welcher ferner mindestens eines umfasst, das aus der Gruppe ausgewählt ist, die aus einer Lösung, einem Reagenz und einem Enzym besteht.

## Revendications

1. Séquence d'acides aminés isolée comprenant un épitope diabétogène, ledit épitope diabétogène comprenant la séquence EEQLRELRRQ (SEQ ID N° 1).

2. Séquence d'acides aminés isolée selon la revendication 1, dans laquelle ladite séquence d'acides aminés comprend la SEQ ID N° 4.

3. Séquence d'acides aminés isolée selon la revendication 1 ou la revendication 2, liée à une protéine transporteuse, une protéine non transporteuse, une macromolécule ou un support.

4. Séquence d'acides aminés isolée selon la revendication 3, liée à un support, ledit support comprenant l'un au moins parmi une bille, une plaque, une boîte, un couvre-objet, une lamelle, une plaque de dosage multi-puits et une biopuce.

5. Séquence nucléotidique codant pour un épitope diabétogène, dans laquelle ledit épitope diabétogène est constitué par la séquence d'acides aminés définie par EEQLRELRRQ (SEQ ID N°1) ou comprend la séquence d'acides aminés définie par la SEQ ID N° 4.

6. Séquence nucléotidique selon la revendication 5, dans laquelle ledit épitope diabétogène comprend la protéine définie par la SEQ ID N° 4.

7. Séquence nucléotidique selon la revendication 5, comprenant une ou plusieurs séquences régulatrices.

8. Séquence nucléotidique selon la revendication 5, comprenant un vecteur de clonage.

9. Séquence nucléotidique complémentaire de la séquence nucléotidique selon la revendication 5.

10. Anticorps isolé capable de se lier à au moins un épitope diabétogène comprenant la séquence EEQLRELRRQ (SEQ ID N°1).

11. Anticorps isolé selon la revendication 10, lequel anticorps comprend un anticorps monoclonal.

12. Anticorps isolé selon la revendication 10, lequel anticorps comprend un anticorps IgG.

13. Anticorps isolé selon la revendication 10, lequel anticorps est produit dans le sérum d'un animal non humain.

14. Anticorps isolé selon la revendication 13, où ledit animal est un animal diabétique.

15. Kit comprenant l'un au moins des éléments suivants :
a) un épitope diabétogène comprenant la séquence EEQLRELRRQ (SEQ ID N° 1),
b) une protéine ou un peptide comprenant un épitope diabétogène, ladite protéine ou ledit peptide comprenant la séquence EEQLRELRRQ (SEQ ID N° 1) ou la SEQ ID N°4,
c) une protéine non transporteuse ou une macromolécule comprenant l'épitope diabétogène EEQLRELRRQ (SEQ ID N° 1) ou la SEQ ID N° 4,
d) un support comprenant l'épitope diabétogène EEQLRELRRQ (SEQ ID N° 1) ou la SEQ ID N° 4,
e) une séquence nucléotidique codant pour un épitope diabétogène constitué par la séquence d'acides aminés définie par EEQLRELRRQ (SEQ ID N° 1) ou comprenant la séquence d'acides aminés définie par la SEQ ID N° 4, et
f) une séquence nucléotidique complémentaire d'une séquence nucléotidique codant pour un épitope diabétogène, constituée par la séquence d'acides aminés définie par EEQLRELRRQ (SEQ ID N°1) ou comprenant la séquence d'acides aminés définie par la SEQ ID N° 4.

16. Kit selon la revendication 15, comprenant en plus au moins un élément choisi dans le groupe constitué par une bille, une plaque, une boîte, un couvre-objet, une lamelle, une plaque de dosage multi-puits, une biopuce.

17. Kit selon la revendication 15, comprenant en plus au moins un anticorps primaire capable de se lier à l'épitope diabétogène EEQLRELRRQ (SEQ ID N° 1) ou à une protéine comprenant l'épitope diabétogène EEQLRELRRQ (SEQ ID N° 1).

18. Kit selon la revendication 17, comprenant en plus au moins un anticorps secondaire capable de se liesr audit au moins un anticorps primaire.

19. Kit selon la revendication 17, comprenant en plus au moins un élément choisi dans le groupe constitué par une solution, un réactif, une enzyme.
